# EUROPÄISCHE PATENTSCHRIFT

(11) **EP 1 945 775 B1**
(45) Veröffentlichungstag und Bekanntmachung des Hinweises auf die Patenterteilung: **09.03.2011**
(21) Anmeldenummer: 06806607.5
(22) Anmeldetag: 30.10.2006
(51) Int. Cl.: C12N 15/82, C07K 14/415

(54) **VERFAHREN ZUR HERSTELLUNG VON GAMMA-LINOLENSÄURE UND/ODER STEARIDONSÄURE IN TRANSGENEN BRASSICACEAE UND LINACEAE**
METHOD FOR THE PRODUCTION OF GAMMA-LINOLENIC ACID AND/OR STEARIDONIC ACID IN TRANSGENIC BRASSICACEAE AND LINACEAE
PROCEDE POUR PRODUIRE DE L'ACIDE GAMMA-LINOLENIQUE ET/OU DE L'ACIDE STEARIDONIQUE DANS DES BRASSICACEES ET LINACEES TRANSGENIQUES

(30) Priorität: 02.11.2005 DE 102005052551
(43) Veröffentlichungstag der Anmeldung: 23.07.2008
(73) Patentinhaber: BASF Plant Science GmbH, 67056 Ludwigshafen (DE)
(72) Erfinder: NAPIER, Johnathan, A., Preston, Hertfordshite SG4 7TR (GB); SAYANOVA, Olga, Harpenden, Herts AL5 2JQ (GB); VENEGAS CALERON, Monica, Harpenden AL5 5pNX Herts (GB)
(86) Internationale Anmeldenummer: PCT/EP2006/010408
(87) Internationale Veröffentlichungsnummer: WO 2007/051577

(56) Entgegenhaltungen:
- WO-A-2005/021761
- WO-A-2005/102310
- OLGA SAYANOVA ET AL: "Identification of Primula front-end desaturases with distinct n-6 or n-3 substrate preferences" PLANTA ; AN INTERNATIONAL JOURNAL OF PLANT BIOLOGY, SPRINGER-VERLAG, BE, Bd. 224, Nr. 6, 14. Juni 2006 (2006-06-14), Seiten 1269-1277, XP019460661 ISSN: 1432-2048
- SAYANOVA O ET AL: "DELTA<6>-Unsaturated fatty acids in species and tissues of the Primulaceae" PHYTOCHEMISTRY, PERGAMON PRESS, GB, Bd. 52, Nr. 3, Oktober 1999 (1999-10), Seiten 419-422, XP004291014 ISSN: 0031-9422
- SAYANOVA O V ET AL: "Identification of Primula fatty acid DELTA<6>-desaturases with n-3 substrate preferences" FEBS LETTERS, ELSEVIER, AMSTERDAM, NL, Bd. 542, Nr. 1-3, 8. Mai 2003 (2003-05-08), Seiten 100-104, XP004422731 ISSN: 0014-5793
- HONG HAIPING ET AL: "High-level production of gamma-linolenic acid in Brassica juncea using a DELTA6 desaturase from Pythium irregulare" PLANT PHYSIOLOGY, AMERICAN SOCIETY OF PLANT PHYSIOLOGISTS, ROCKVILLE, MD, US, Bd. 129, Nr. 1, Mai 2002 (2002-05), Seiten 354-362, XP002207405 ISSN: 0032-0889

### SEQUENCE LISTING

<110> Rothamsted Research Ltd.
<120> verfahren zur Herstellung von gamma-Linolensäure und/oder Stearidonsäure in transgenen Brassicaceae und Linaceae
<130> 2005/1063
<140> PF 57260
   <141>
<160> 12
<170> PatentIn version 3.1
<210>
   <211> 1350
   <212> DNA
   <213> Primula lutoides
<220>
   <221> CDS
   <222> (1)..(1350)
   <223> Delta-6-Desaturase
<400> 1
<210> 2
   <211> 449
   <212> PRT
   <213> Primula lutoides
<400> 2
   Met Ala Asn Lys Ser Gln Thr Gly Tyr Ile Thr Ser Ser Glu Leu Glu
   1               5                   10                  15
   Thr His Asn Lys Ala Gly Asp Leu Trp Ile Ser Ile His Gly Gln Val
               20                  25                  30
   Tyr Asp Val Ser Ser Trp Ala Gly Leu His Pro Gly Gly Thr Ala Pro
           35                  40                  45
   Leu Leu Ala Leu Ala Gly His Asp Val Thr Asp Ala Phe Leu Ala Tyr
       50                  55                  60
   His Pro Pro Ser Thr Ala Arg Leu Leu Pro Pro Leu Ser Thr His Leu
   65                  70                  75                  80
   Leu Leu Gln His His Ser Val Ser Pro Thr Ser Ser Asp Tyr Arg Ser
                   85                  90                  95
   Leu Leu Asp Asn Phe His Lys Leu Gly Leu Phe Arg Ala Arg Gly His
               100                 105                 110
   Thr Ala Tyr Ala Thr Phe Val Ile Met Ile Ala Met Phe Leu Ala Ser
           115                 120                 125
   Val Thr Gly Val Leu Cys Ser Asp Lys Ala Trp Val His Leu Ala Ser
       130                 135                 140
   Gly Gly Ala Met Gly Phe Ala Trp Ile Gln Cys Gly Trp Ile Gly His
   145                 150                 155                 160
   Asp Ser Gly His Tyr Arg Ile Met Ser Gly Glu Lys Trp Asn Arg Phe
                   165                 170                 175
   Ala Gln Ile Leu Ser Thr Asn Cys Leu Gln Gly Ile Ser Ile Gly Trp
               180                 185                 190
   Trp Lys Trp Asn His Asn Ala His His Ile Ala Cys Asn Ser Leu Asp
           195                 200                 205
   Tyr Asp Pro Asp Leu Gln Tyr Ile Pro Leu Leu Val Val Ser Pro Lys
       210                 215                 220
   Phe Phe Asn Ser Leu Thr Ser Arg Phe Tyr Asp Lys Lys Leu Asn Phe
   225                 230                 235                 240
   Asp Gly Val Ser Arg Phe Leu Val Cys Tyr Gln His Trp Thr Phe Tyr
                   245                 250                 255
   Pro Val Met Cys Val Ala Arg Leu Asn Met Ile Ala Gln Ser Phe Ile
               260                 265                 270
   Met Leu Phe Ser Ser Arg Glu Val Ala Gln Arg Val Gln Gly Ile Phe
           275                 280                 285
   Gly Leu Ala Val Phe Trp Val Trp Phe Pro Leu Leu Leu Ser Cys Leu
       290                 295                 300
   Pro Asn Trp Gly Glu Arg Ile Met Phe Leu Leu Ala Ser Tyr Ser Val
   305                 310                 315                 320
   Thr Gly Ile Gln His Val Gln Phe Ser Leu Asn His Phe Ser Ser Asp
                   325                 330                 335
   Val Tyr Val Gly Pro Pro Val Gly Asn Asp Trp Phe Lys Lys Gln Thr
               340                 345                 350
   Ala Gly Thr Leu Asn Ile Ser Cys Pro Ala Trp Met Asp Trp Phe His
           355                 360                 365
   Gly Gly Leu Gln Phe Gln Val Glu His His Leu Phe Pro Arg Met Pro
       370                 375                 380
   Arg Gly Gln Phe Arg Lys Ile Ser Pro Phe Val Arg Asp Leu Cys Lys
   385                 390                 395                 400
   Lys His Asn Leu Pro Tyr Asn Ile Ala Ser Phe Thr Lys Ala Asn Val
                   405                 410                 415
   Leu Thr Leu Met Thr Leu Arg Asn Thr Ala Val Glu Ala Arg Asp Leu
               420                 425                 430
   Ser Asn Pro Ile Pro Lys Asn Met Val Trp Glu Ala Val Gln Thr Leu
           435                 440                 445
   Gly
<210> 3
   <211> 1353
   <212> DNA
   <213> Primula cortusoides
<220>
   <221> CDS
   <222> (1)..(1353)
   <223> Delta-6-Desaturase
<400> 3
   atg gcc aac cca tca aaa aac agt tac att tcc gtc tca gac ctc aaa      48
   Met Ala Asn Pro Ser Lys Asn Ser Tyr Ile Ser Val Ser Asp Leu Lys
   1               5                   10                  15
   acc cac aac aag ccc gga gac ctc tgg ata tcc atc cac ggc caa gtc      96
   Thr His Asn Lys Pro Gly Asp Leu Trp Ile Ser Ile His Gly Gln Val
               20                  25                  30
   tac gac gtc tct gcg tgg gcg cca cgc cac cct ggc ggc ctc cct ctc     144
   Tyr Asp Val Ser Ala Trp Ala Pro Arg His Pro Gly Gly Leu Pro Leu
           35                  40                  45
   ctc ctc tct cac ggc ggt cat gac gtc acg gat gcc ttc ctc gcc tac     192
   Leu Leu Ser His Gly Gly His Asp Val Thr Asp Ala Phe Leu Ala Tyr
       50                  55                  60
   cac ccc ccc tcg gtt tcc cgc ctc ctc cct tct ctc tct acc tac ctc     240
   His Pro Pro Ser Val Ser Arg Leu Leu Pro Ser Leu Ser Thr Tyr Leu
   65                  70                  75                  80
   cgc ctc gaa aac cac tcc gtc tcc gcc ccc tcc tcc gac tac cgc acc     288
   Arg Leu Glu Asn His Ser Val Ser Ala Pro Ser Ser Asp Tyr Arg Thr
                   85                  90                  95
   ctc ctt tcc cat ttc gac aac ctc ggc ctc ttc cac acc aag ggc cac     336
   Leu Leu Ser His Phe Asp Asn Leu Gly Leu Phe His Thr Lys Gly His
               100                 105                 110
   acc att ctc gcc act ttc gtc atc atg att gcc agt atc ctc ttc tgc      384
   Thr Ile Leu Ala Thr Phe Val Ile Met Ile Ala Ser Ile Leu Phe Cys
           115                 120                 125
   cta tgt ggg atc ttc ctc agt act agt ttc tgg gtc cac ttg gcg agc      432
   Leu Cys Gly Ile Phe Leu Ser Thr Ser Phe Trp Val His Leu Ala Ser
       130                 135                 140
   ggc gtc ctg att ggg ttc gcc tgg atc cag tgc ggg tgg ctc ggg cac      480
   Gly Val Leu Ile Gly Phe Ala Trp Ile Gln Cys Gly Trp Leu Gly His
   145                 150                 155                 160
   gac tcc gga cat tac aaa ata aca tcc ggt aaa aaa tcc aac cgc ttc      528
   Asp Ser Gly His Tyr Lys Ile Thr Ser Gly Lys Lys Ser Asn Arg Phe
                   165                 170                 175
   gct cag gtt ctg gtc gga aac tgc ttc gcg ggg att agc atc gag tgg      576
   Ala Gln Val Leu Val Gly Asn Cys Phe Ala Gly Ile Ser Ile Glu Trp
               180                 185                 190
   tgg aaa tgg aac cac aac gct cac cac acc tct tgc aac agc ctc gac      624
   Trp Lys Trp Asn His Asn Ala His His Thr Ser Cys Asn Ser Leu Asp
           195                 200                 205
   cac gac ccc gac ctc caa tac att ccc ttc ttg gtc gtt tcc tcc aag      672
   His Asp Pro Asp Leu Gln Tyr Ile Pro Phe Leu Val Val Ser Ser Lys
       210                 215                 220
   ttc ttc act tcc atg atc act tct cgt ttc tac aac aaa aag ctg aat      720
   Phe Phe Thr Ser Met Ile Thr Ser Arg Phe Tyr Asn Lys Lys Leu Asn
   225                 230                 235                 240
   ttc aat gct atg tcg agg ttt tta gtc agc tat cag cat tgg tcg ttt      768
   Phe Asn Ala Met Ser Arg Phe Leu Val Ser Tyr Gln His Trp Ser Phe
                   245                 250                 255
   tat ccg gtt atg tgt ctc gcg agg gtc aac atg ttt ctg cag tcg ctt      816
   Tyr Pro Val Met Cys Leu Ala Arg Val Asn Met Phe Leu Gln Ser Leu
               260                 265                 270
   gtc ttc ctt ttt ttc aat aag gag gtg caa aat agg gtt caa gag att      864
   Val Phe Leu Phe Phe Asn Lys Glu Val Gln Asn Arg Val Gln Glu Ile
           275                 280                 285
   cta ggg ata gct gtg ttc tgg gtt tgg ttt ccg ctc gta gtt tct tcc      912
   Leu Gly Ile Ala Val Phe Trp Val Trp Phe Pro Leu Val Val Ser Ser
       290                 295                 300
   ctt cct aat tgg ggt gag aga ata atg ttt ctg gtt gcg agc ttc tct      960
   Leu Pro Asn Trp Gly Glu Arg Ile Met Phe Leu Val Ala Ser Phe Ser
   305                 310                 315                 320
   att aca gga atc caa cag gtg cag ttt agc gta aac cat ttt tcg tcg     1008
   Ile Thr Gly Ile Gln Gln Val Gln Phe Ser Val Asn His Phe Ser Ser
                   325                 330                 335
   gat gtc tac gtc ggc cct ccg atg gaa aac gat tgg ttt gaa aaa cag     1056
   Asp Val Tyr Val Gly Pro Pro Met Glu Asn Asp Trp Phe Glu Lys Gln
               340                 345                 350
   act gcc ggg acg ctc aac ata tcg tgc ccg acg tgg atg gat tgg ttc     1104
   Thr Ala Gly Thr Leu Asn Ile Ser Cys Pro Thr Trp Met Asp Trp Phe
           355                 360                 365
   cat ggc ggg ttg cag ttt caa atc gag cac cac ttg ttc ccg cgg atg     1152
   His Gly Gly Leu Gln Phe Gln Ile Glu His His Leu Phe Pro Arg Met
       370                 375                 380
   ccg agg agt caa ctt aga aag atc tct cct ttt gtt aag gat ttg tgt     1200
   Pro Arg Ser Gln Leu Arg Lys Ile Ser Pro Phe Val Lys Asp Leu Cys
   385                 390                 395                 400
   aaa aaa cat aac ttg cct tac aag atc gcg tct ttt aca acg gcc aat     1248
   Lys Lys His Asn Leu Pro Tyr Lys Ile Ala Ser Phe Thr Thr Ala Asn
                   405                 410                 415
   gtg ttg atg ctt agg act ctg aga aat gtt gct att aag gct cgg gac     1296
   Val Leu Met Leu Arg Thr Leu Arg Asn Val Ala Ile Lys Ala Arg Asp
               420                 425                 430
   ctt tct aat ccg atc ccg aag aat ttg gtg tgg gaa gcc ttc cac aca     1344
   Leu Ser Asn Pro Ile Pro Lys Asn Leu Val Trp Glu Ala Phe His Thr
           435                 440                 445
   cac gga taa                                                         1353
   His Gly
       450
<210> 4
   <211> 450
   <212> PRT
   <213> Primula cortusoides
<400> 4 450
<210> 5
   <211> 27
   <212> DNA
   <213> unknown
<220>
   <223> artificial sequence
   <220>
   <221> misc_feature
   <222> (1)..(27)
   <223>
<400> 5
<400> 5
   ggtaccatgg ccaacccatc aaaaaac 27
<210> 6
   <211> 27
   <212> DNA
   <213> unknown
<220>
   <223> artificial sequence
   <220>
   <221> misc_feature
   <222> (1)..(27)
   <223>
<400> 6
   ccttccacac acacggataa gagctcc 27
<210> 7
   <211> 26
   <212> DNA
   <213> unknown
<220>
   <223> artificial sequence
   <220>
   <221> misc_feature
   <222> (1)..(26)
   <223>
<400> 7
   ggtaccatgg ctaacaaatc caaac 26
<210> 8
   <211> 26
   <212> DNA
   <213> unknown
<220>
   <223> artificial sequence
   <220>
   <221> misc_feature
   <222> (1)..(26)
   <223>
<400> 8
   ctgttcaaac tctcgggtga ggagct 26
<210> 9
   <211> 30
   <212> DNA
   <213> unknown
<220>
   <223> artificial sequence
   <220>
   <221> misc_feature
   <222> (1)..(30)
   <223>
<400> 9
   ggatccacca tggccaaccc atcaaaaaac 30
<210> 10
   <211> 28
   <212> DNA
   <213> unknown
<220>
   <223> artificial sequence
   <220>
   <221> misc_feature
   <222> (1)..(28)
   <223>
<400> 10
   ccttccacac acacggataa ggtctaga 28
<210> 11
   <211> 29
   <212> DNA
   <213> unknown
<220>
   <223> artificial sequence
   <220>

   <221> misc_feature
   <222> (1)..(29)
   <223>
<400> 11
   ggatccacca tggctaacaa atctcaaac 29
<210> 12
   <211> 28
   <212> DNA
   <213> unknown
<220>
   <223> artificial sequence
   <220>
   <221> misc_feature
   <222> (1)..(28)
   <223>
<400> 12
   ctgttcaaac tctcgggtga ggtctaga 28

## Beschreibung

Die vorliegende Erfindung betrifft die Herstellung von γ-linolensäure (18:3 ^{Δ6,9,12}) oder Stearidonsäure (18:4 ^{Δ6,9,12,15}) oder y-Linolensäure (18:3 ^{Δ6,9,12}) und Stearidonsäure (18:4 ^{Δ6,9,12,15}) in transgenen Pflanzen der Familie Brassicaceae, wobei die transgenen Pflanzen mindestens 10 Gew-% Ölsäure bezogen auf den Gesamtfettsäuregehalt enthalten und durch die Aktivität der im Verfahren verwendeten Δ-6-Desaturasen einen erhöhten Gehalt an Δ-6-C₁₈-Fettsäuren aufweisen.

Außerdem betrifft die Erfindung neue Nukleinsäuresequenzen, die für die im Verfahren verwendeten Δ-6-Desaturasen codieren, Genkonstrukte enthaltend diese Nukleinsäuresequenzen, einen Vektor und transgene Pflanzen enthaltend mindestens eine Nukleinsäuresequenz bzw. ein Genkonstrukt.

Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet.

Mehrfach ungesättigte langkettige ω-3-Fettsäuren wie Eicosapentaensäure (EPA) oder Docosahexaensäure (DPA) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättiger langkettiger Fettsäuren und speziell an ω-3-Fettsäuren.

So werden beispielsweise mehrfach ungesättigte Fettsäuren Babynahrung zur Erhöhung des Nährwertes zugesetzt, sowie zur ungehinderten Entwicklung des Säuglings.. Hauptsächlich werden die verschiedenen Fettsäuren und Triglyceride aus Mikroorganismen wie Mortierella oder aus Öl-produzierenden Pflanzen wie Soja, Raps, Sonnenblume und weiteren gewonnen, wobei sie in der Regel in Form ihrer Triacylglyceride anfallen. In höheren Pflanzen treten allerdings keine langkettigen ungesättigten Fettsäuren auf. Die langkettigen Fettsäuren stammen zum größten Teil aus Fischöl bzw. aus der Fermentation von entsprechenden Algen (z.B. Thraustochytrium) bzw. Pilzen (z.B. Mortierella). Die freien Fettsäuren werden vorteilhaft durch Verseifung hergestellt.

Je nach Anwendungszweck sind Öle mit gesättigten oder ungesättigten Fettsäuren bevorzugt, so sind z.B. in der humanen Ernährung Lipide mit ungesättigten Fettsäuren speziell mehrfach ungesättigten Fettsäuren bevorzugt, da sie einen positiven Einfluss auf den Cholesterinspiegel im Blut und damit auf die Möglichkeit einer Herzerkrankung haben. Sie finden in verschiedenen diätischen Lebensmitteln oder Medikamenten Anwendung.

Die beiden Fettsäuren y-Linolensäure und Stearidonsäure, besonders die Stearidonsäure, haben vielfach beschrieben positive Auswirkungen auf den Hautstoffwechsel (z.B., Akne, Sonnenschäden) und auf die Verlangsamung der Hautalterungsprozesse, bis hin zu vorbeugender und heilender Wirkung bei den verschiedensten Entzündungsprozessen im Körper. Auch in der Begleittherapie von Prostata- und Darmkrebs und in Therapie und Vorbeugung von neurologischen Störungen (z.B. Dyspraxie, Schizophrenie) finden sie Anwendung. Deswegen spielen diese Fettsäuren auch in der Kosmetikindustrie und bei Nahrungsmittelzusätzen eine große Rolle. Bislang werden y-Linolensäure- und Stearidonsäure -reiche Öl vor allem aus Arten der Gattung Echium gewonnen.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine delta - 15-Desaturase in WO 94/11516 wird eine Δ-12-Desaturase beansprucht. Weitere Desaturasen werden beispielsweise in EP-A-0 550162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ - 6-Desaturasen werden in WO 93/06712, US 5,614,393, US5614393, WO 96/21022, WO0021557 und WO 99/27111 beschrieben und auch die Anwendung zur Produktion in transgenen Organismen beschrieben wie in WO9846763 WO9846764, WO9846765. Dabei wird auch die Expression verschiedener Desaturasen wie in WO9964616 oder WO9846776 und Bildung polyungesättigter Fettsäuren beschrieben und beansprucht.

Nach wie vor besteht daher ein großer Bedarf an neuen und besser geeigneten Genen, die für Enzyme kodieren, die an der Biosynthese ungesättigter Fettsäuren beteiligt sind und es ermöglichen, bestimmte Fettsäuren spezifisch in einem technischen Maßstab herzustellen, ohne dass unerwünschte Nebenprodukte entstehen. Bei der Auswahl von Biosynthesegenen sind vor allem zwei Merkmale besonders wichtig. Zum einen besteht nach wie vor ein Bedarf an verbesserten Verfahren zur Gewinnung möglichst hoher Gehalte an polyungesättigten Fettsäuren. Zum anderen sollten die eingesetzten Enzyme hoch spezifisch für ein bestimmtes Substrat sein, da möglichst keine ungewünschten Nebenprodukte entstehen dürfen, die möglicherweise negative oder bisher nicht erforschte physiologische Effekte in der Nahrungsmittelanwendung haben.

Um eine Anreicherung der Nahrung und des Futters mit diesen spezifisch hergestellten, mehrfach ungesättigten Fettsäuren zu ermöglichen, besteht daher ein großer Bedarf an einem einfachen, kostengünstigen Verfahren zur Herstellung dieser mehrfach ungesättigten Fettsäuren mit Hilfe von möglichst spezifischen Enzymen, die an der Fettsäurebiosynthese beteiligt sind.

Es bestand daher die Aufgabe ein neues Verfahren für die Herstellung von y-Linolensäure und/oder Stearidonsäure in einer Pflanze zu entwickeln, dass möglichst spezifisch die Synthese dieser Fettsäuren erlaubt. Diese Aufgabe wurde durch das vorliegende Verfahren zur Herstellung von γ-Linolensäure (18:3 ^{Δ6,9,12}) oder Stearidonsäure (18:4 ^{Δ6,9,12,15}) oder γ-Linolensäure (18:3 ^{Δ6,9,12}) und Stearidonsäure (18:4 ^{Δ6,9,12,15}) in transgenen Pflanzen der Familie Brassicaceae oder Linaceae gelöst, dadurch gekennzeichnet, dass die transgenen Pflanzen mindestens 10 Gew-% Ölsäure bezogen
auf den Gesamtfettsäuregehalt enthalten und das folgende Verfahrensschritte umfassend:
a) Einbringen einer Nukleinsäuresequenz in die Ölpflanze, die für die unten näher definierte Δ-6-Desaturase aus einer Primula-Art codiert und als Substrat α-Linolensäure gegenüber Linolsäure bevorzugt, und
b) Expression der durch die Nukleinsäure codierten Δ-6-Desaturase in der transgenen Pflanze, und
c) Anzucht und Ernte der Pflanzen.

Vorteilhaft enthalten die transgenen Ölpflanzen der Familie der Brassicaceae oder Linaceae mindestens 11, 12, 33, 14 oder 15 Gew-% Ölsäure, vorteilhaft mindestens 16, 17, 18, 19 oder 20 Gew-% Ölsäure bezogen auf den Gesamtfettsäuregehalt, besonders vorteilhaft mindestens, 25, 30, 35, 40, 45, 50, 55 oder 60 Gew-% Ölsäure bezogen auf den Gesamtfettsäuregehalt, ganz besonders vorteilhaft mindestens 61, 62, 63, 64, 65, 66, 67, 68, 69 oder 70 Gew-% Ölsäure bezogen auf den Gesamtfettsäuregehalt oder mehr. Für das erfindungsgemäße Verfahren vorteilhafte Pflanzen weisen außerdem einen bevorzugten Palmitinsäuregehalt von mindestens 9, 10, 11, 12, 13, 14 oder 15 Gew.-%, vorteilhaft von 20, 21, 22, 23, 24 oder 25 Gew-%, besonders vorteilhaft von 26, 27, 28, 29 oder 30 Gew-% bezogen auf den Gesamtfettsäuregehalt. Weitere vorteilhafte Pflanzen weisen einen Linolsäuregehalt von mindestens 20, 25, 30, 35, 40, 45 oder 50 Gew-%, vorteilhaft von 55, 60, 65, 70 oder 75 Gew-% bezogen auf den Gesamtfettsäuregehalt auf. Andere vorteilhafte Pflanzen weisen einen α-Linolensäuregehalt von mindestens 10, 15, 20, 25 oder 30 Gew.%, vorteilhaft von 35, 40, 45 oder 50 Gew-%, besonders vorteilhaft von 55, 60, 65 oder 70 Gew.-% bezogen auf den Gesamtfettsäuregehalt auf, Vorteilhafte Pflanzen können auch Kombinationen der vorgenannten Fettsäuren aufweisen, wobei der Gesamtfettsäuregehalt 100 Gew-% beträgt.

Die im Verfahren verwendeten transgenen Pflanzen der Familie Brassicaceae oder Linaceae haben vorteilhaft einen Gesamtölgehalt im Samen von mindestens 20, 25 oder 30 Gew-%, vorteilhaft um mindestens 35 oder 40 Gew-%, besonders vorteilhaft um mindestens 45 oder 50 Gew-%, ganz besonders vorteilhaft von mindestens 55 Gew-% bezogen auf das Gesamtgewicht des Samens.

Im Verfahren bevorzugte Ölfruchtpflanzen produzieren Öle, Lipide und/oder freie Fettsäuren, die weniger als 4, 3, 2 oder 1 Gew.%, vorteilhaft weniger als 0,9; 0,8; 0,7; 0,6 oder 0,5 Gew.%, besonders vorteilhaft weniger als 0,4; 0,3; 0,2; 0,1 oder 0,09 Gew.-% oder weniger Myristinsäure enthalten. Weitere vorteilhafte Ölfruchtpflanzen enthalten weniger als 5,4 oder 3 Gew.-% Palmitinsäure und/oder weniger als 2; 1,5 oder 1 Gew.% Stearinsäure.

Vorteilhafte Ölpflanzen sollten neben einem hohen Ölgehalt im Samen auch einen geringen Proteinanteil im Samen haben. Dieser Proteinanteil sollte möglichst geringer als 30, 25 oder 20 Gew.%, vorteilhaft geringer als 19, 18, 17, 16 oder 15 Gew.-% sein.

Für das erfindungsgemäße Verfahren sind prinzipiell alle Gattungen der Familie Brassicaceae (ca. 380 Gattungen weltweit) mit ihren Unterfamilien Arabideae, Brassiceae, Chamireae, Cremolobeae, Heliophileae, Hesperideae, Lepidieae, Prynglea, Schizopetaleaee, Sisymbrieae, Stenopetaleae und Thelypodieae oder der Familie Linaceae (9 Gattungen weltweit) geeignet. Bevorzugt werden für das erfindungsgemäße Verfahren Gattungen der Familien Brassicaceae oder Linaceae ausgewählt aus der Gruppe bestehend aus. Alliaria, Alyssoides, Arabis, Armoracia, Barbarea, Berteroa, Brassica, Camelina, Capsella, Cardamine, Cardaria, Cheiranthus, Crambe, Dentaria, Diplotaxis, Erophila, Erysimum, Iberis, Lepidium, Lunaria, Nasturtium, Raphanus, Rorippa, Schivereckia, Sinapis, Sisymbrium, Thlaspi, Turritis, Anisadenia, Cliococca, Durandea, Hebepetalum, Hesperolinon, Hugonia, Indorouchera, Linum, Philbornea, Radiola, Reinwardtia, Roucheria, Sclerolinon und Tirpitzia.

Besonders bevorzugt für das erfindungsgemäße Verfahren werden die Gattungen und Arten ausgewählt aus der Gruppe bestehend aus Alliaria petiolata, Alyssoides utriculata, Arabis caucasica, Arabis procurrens, Arabis turrita, Armoracia rusticana, Barbarea intermedia, Barbaraea vulgaris, Berteroa incana, Brassica napus, Brassica napus ssp. rapifera, Brassica napus ssp. napus, Brassica nigra, Brassica oleracea, Brassica rapa, Brassica rapa ssp. oleifera, Brassica rapa ssp. rapa, Brassica sativus, Camelina sativa, Capsella bursa-pastoris, Cardamine amara, Cardamine bulbifera, Cardamine hirsuta, Cardamine pratensis, Cardaria draba, Cheiranthus cheiri, Crambe abyssinica, Dentaria bulbifera, Diplotaxis tenuifolia, Erophila vema, Erophila vema agg, Erysimum bicolor, Erysimum cheiranthoides, Iberis spec, Lepidium campestre, Lepidium sativum, Lepidium virginicum, Lunaria annua, Lunaria rediviva, Nasturtium officinale, Raphanus raphanistrum, Rorippa pyrenaica, Schivereckia podolica, Sinapis alba, Sinapis arvensis, Sisymbrium officinale, Thlaspi arvense, Thlaspi perfoliatum, Turritis glabra, Linum alpinum , Linum austriacum, Linum catharticum, Linum flavum, Linum grandiflorum Linum hirsutum , Linum leonii, Linum maritimum , Linum ockendonii, Linum perenne, Linum tenuifolium, Linum viscosum und Linum usitatissimum.

Besonders vorteilhaft werden die im Verfahren verwendeten Pflanzen ausgewählt aus der Gruppe der Ölpflanzen ausgewählt aus der Gruppe bestehend aus den Gattungen und Arten Brassica campestris, Brassica napus, Brassica rapa, Brassica juncea, Camelina sativa, Crambe abyssinica und Linum usitatissimum. Ganz besonders bevorzugt werden im erfindungsgemäßen Verfahren die Gattungen und Arten Brassica campestris, Brassica napus, Brassica rapa, Brassica juncea, Camelina sativa und Linum usitatissimum verwendet.

Im erfindungsgemäßen Verfahren werden Nukleinsäuresequenzen verwendet, die für eine Δ-6-Desaturase aus Primula-Arten codieren, ausgewählt ist aus der Gruppe bestehend aus:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide codieren, die auf Aminosäureebene mindestens 95% identität mit SEQ ID NO: 2 aufweisen und eine Δ-6-Desaturaseaktivität haben. Die Δ-6-Desaturasen haben eine Substrat spezifität, fur α-Lindensaüre, die um mehr als 20 fach höher ist als die fur linolsaüre.

Vorteilhaft werden im Verfahren zur Expression der unter (a), (b) und (c) genannten Nukleinsäuresequenzen diese funktionell mit einem Promotor oder Terminator oder Promotor und Terminator verbunden.

Im erfindungsgemäßen Verfahren zur Herstellung von γ-Linolensäure und/oder Stearidonsäure in einer transgenen Pflanze der Familie Brassicaceae oder Linaceae wird durch die Aktivität der Δ-6-Desaturase der Gehalt an Δ-6-C₁₈-Fettsäuren gegenüber der nicht-transgenen Ausgangspflanze (wildtyp) erhöht. Unter dem Begriff "erhöht" im Sinne der Erfindung ist zu verstehen, dass der Gehalt an Δ-6-C₁₈-Fettsäuren (γ-Linolensäure und/oder Stearidonsäure) gegenüber dem Wildtyp um mindestens 25, 30, 35, 40, 45 oder 50 %, vorteilhaft um mindestens 55, 60, 65, 70, 75, 80, 85, 90, 95 oder 100 %, besonders vorteilhaft um mindestens 105, 110, 115, 120, 125, 130, 135, 140, 145 oder 150 % oder mehr erhöht wird.

Vorteilhaft weist die im Verfahren verwendete Δ-6-Desaturase eine Substratspezifität gegenüber α-Linolensäure auf, die um mehr als 25, 30, 35 oder 40fach höher ist als die Substratspezifität gegenüber Linolsäure. In einer besonders vorteilhaften Ausführungsform der Erfindung besitzt die Δ-6-Desaturase eine ausschließliche Substratspezifität gegenüber α-Linolensäure, das heißt das Enzym erkennt als Substrat nur α-Linolensäure nicht jedoch Linolsäure. Dies führt zu einer vorteilhaften Synthese von ungesättigten Fettsäuren des ω-3-Syntheseweges, während Linolsäure als Startsubstrat des ω-6-Syntheseweges von der Desaturase nicht umgesetzt wird.

Vorteilhaft werden γ-Linolensäure und/oder Stearidonsäure als Produkte der enzymatischen Aktivität der Δ-6-Desaturase im Verfahren im Samen der Ölpflanzen angereichert.

Die im Verfahren hergestellten Fettsäuren γ-Linolensäure und/oder Stearidonsäure fallen vorteilhaft als freie Fettsäuren oder bevorzugt in Form von Estern bevorzugt in Form der Triglyceride an.

Vorteilhaft werden im erfindungsgemäßen Verfahren transgene Pflanzen der Familie Brassicaceae oder Linaceae verwendet. Diese Pflanzen enthalten die im erfindungsgemäßen Verfahren synthetisierten mehrfach ungesättigten Fettsäuren γ-Linolensäure und/oder Stearidonsäure und können vorteilhaft direkt vermarktet werden ohne dass, die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Pflanzen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Die im Verfahren hergestellten PUFAs fallen in den Pflanzen vorteilhaft in Form ihrer Öle, Lipide oder Fettsäuren oder Fraktionen davon an.

Öle, Lipide, Fettsäuren und/oder Fettsäurezusammensetzungen, die durch das Mischen der hergestellten Öle, Lipide oder Fettsäuren mit anderen tierischen, mikrobiellen oder pflanzlichen Ölen hergestellt wurden, können in Futtermitteln, Nahrungsmitteln, Kosmetika oder Pharmazeutika verwendet wurden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, α-Linolensäure, γ-Linolensäure und/oder Stearidonsäure hat. Vorzugsweise ist der Anteil an ungesättigten veresterten Fettsäuren ungefähr 30, mehr bevorzugt ist ein Anteil von 50, noch mehr bevorzugt ist ein Anteil von 60, 70, 80 Gew.% oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren, handelt es sich beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren lassen sich die enthaltenden Fettsäuren beispielsweise über eine Alkalibehandlung beispielsweise wässrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Aus den Pflanzen werden nach Anzucht die Öle, Lipide oder Fettsäuren in üblicherweise gewonnen. Hierzu können wie beschrieben die Pflanzen und/oder deren Samen nach Ernte zunächst aufgeschlossen werden oder direkt verwendet werden. Die Öle, Lipide und/oder Fettsäuren werden vorteilhaft mit geeigneten Lösungsmitteln wie apolare Lösungsmittel wie Hexan oder Ethanol, Isopropanol oder Gemischen wie Hexan/Isopropanol, Phenol/Chloroform/Isoamylalkohol bei Temperaturen zwischen 0°C bis 80°C, bevorzugt zwischen 20°C bis 50°C extrahiert. Die Biomasse wird in der Regel mit einem Überschuss an Lösungsmittel extrahiert beispielsweise einem Überschuss von Lösungsmittel zu Biomasse von 1:4. Das Lösungsmittel wird anschließend beispielsweise über eine Destillation entfernt. Die Extraktion kann auch mit superkritischem CO₂ erfolgen. Nach Extraktion kann die restliche Biomasse beispielsweise über Filtration entfernt werden.

Das so gewonnene Rohöl kann anschließend weiter aufgereinigt werden, beispielsweise in dem Trübungen über das Versetzen mit polaren Lösungsmittel wie Aceton oder Chloroform und anschließender Filtration oder Zentrifugation entfernt werden. Auch eine weitere Reinigung über Säulen ist möglich.

Zur Gewinnung der freien Fettsäuren aus den Triglyceriden werden diese, wie beschrieben, in üblicherweise verseift.

Die so im Verfahren gewonnenen Öle, Lipide und/oder freien Fettsäuren oder die Trigylceride mit einem erhöhten Gehalt an γ-Linolensäure und/oder Stearidonsäure, die nach den oben genannten Verfahren hergestellt wurden, können zur Herstellung von Nahrungsmitteln, Tierfutter, Kosmetika oder Pharmazeutika verwendet werden. Hierzu werden diese den Nahrungsmitteln, dem Tierfutter, den Kosmetika oder Pharmazeutika in üblichen Mengen zugesetzt. In einer anderen Ausführungsform werden die Öle, Lipide und/oder freien Fettsäuren oder die Trigylceride mit einem erhöhten Gehalt an γ-Linolensäure und/oder Stearidonsäure mit anderen tierischen, mikrobiellen oder pflanzlichen Ölen, Lipiden oder Fettsäuren zu Fettsäurezusammensetzungen gemischt. Diese können Nahrungsmitteln, dem Tierfutter, den Kosmetika oder Pharmazeutika in üblichen Mengen zugesetzt werden.

Die oben genannten Verfahren ermöglichen vorteilhaft die Synthese von Fettsäuren oder Triglyceriden mit einem erhöhten Gehalt an Fettsäuren mit Δ-6-Doppelbindungen vorteilhaft an C18-Fettsäuren.

Das oben genannte Verfahren ermöglicht vorteilhaft die Synthese von Fettsäuren oder Triglyceriden mit einem erhöhten Gehalt an Fettsäuren mit Δ-6-Doppelbindungen, wobei als Substrat für die Reaktion der Δ-6-Desaturase bevorzugt Linolsäure und/oder α-Linolensäure vorteilhaft nur α-Linolensäure verwendet wird. Damit ermöglicht das oben genannte Verfahren vorteilhaft besonders die Synthese von γ-Linolensäure und/oder Stearidonsäure bevorzugt nur von Stearidonsäure. Diese spezielle Substratspezifität der erfindungsgemäßen Δ-6-Desaturase unterscheidet sich vorteilhaft von den Substratspezifitäten der Δ-6-Desaturasen des Standes der Technik.

Ein weiterer Erfindungsgegenstand sind somit isolierte Nukleinsäuresequenzen, die für Polypeptide mit Δ-6-Desaturaseaktivität codieren und wobei die codierte Δ-6-Desaturase als Substrat α-Linolensäure gegenüber Linolsäure bevorzugt, ausgewählt aus der Gruppe bestehend aus:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide codieren, die auf Aminosäureebene mindestens 95 % Homologie mit SEQ ID NO: 2 aufweisen und die eine Δ-6-Desaturaseaktivität wie zuvor definiert haben.

Die gefundenen Δ-6-Desaturasen unterscheiden sich von den bereits beschriebenen Δ-6-Desaturasen durch wesentlich unterschiedliche Nukleotid- und Aminosäuresequenzen. Die Primula-Sequenz von P. cortusoides zeigt zu den Δ-6-Desaturase-Sequenzen des Standes der Technik eine 78 %ige Ähnlichkeit auf Aminosäure-Ebene. Die erfindungsgemäße Δ-6-Desaturase-Sequenz von P. lutoides hat eine 92%ige Ähnlichkeit auf Aminosäure-Ebene zu den Sequenzen des Standes der Technik.

Vorteilhaft stammen die erfindungsgemäßen Nukleinsäuresequenzen aus einer Pflanze, bevorzugt aus einer Pflanze der Familie der Primulaceae, besonders bevorzugt aus der Gattung Primula, ganz besonders bevorzugt aus den Gattungen und Arten Primula cortusoides oder Primula lutoides.

Der Begriff "Δ-6-Desaturase" im Sinne der Erfindung umfasst Proteine, die an der Desaturierung von Fettsäuren vorteilhaft von Fettsäuren, die an Position 9 der Fettsäurekette eine Doppelbindung haben, teilnehmen, sowie ihre Homologen, Derivaten oder Analoga.

Bei einer weiteren Ausführungsform kodieren Derivate des erfindungsgemäßen Nukleinsäuremoleküls wieder gegeben in SEQ ID NO: 1 Proteine mit mindestens 95% oder 96% und am stärksten bevorzugt mindestens etwa 97 %, 98 %, 99 % oder mehr Homologie (= Identität) zur vollständigen Aminosäuresequenz SEQ ID NO: 2. Die Homologie wurde über den gesamten Aminosäure- bzw. Nukleinsäuresequenzbereich berechnet. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight 8, Length Weight: 2, Average Match: 2,912 und Average Mismatch: - 2,003.

Die Erfindung umfasst zudem Nukleinsäuremoleküle, die sich von einer der in SEQ ID NO: 1 gezeigten Nukleinsäuresequenz (und Teilen davon) aufgrund des degenerierten genetischen Codes unterscheiden und somit die gleiche Δ-6-Desaturase kodieren wie diejenige, die von der In SEQ ID NO: 1 gezeigten Nukleotidsequenz kodiert wird.

Zusätzlich zu der in SEQ ID NO: 1 gezeigten Δ-6-Desaturase-Nukleotidsequenz erkennt der Fachmann, dass DNA-Sequenzpolymorphismen, die zu Änderungen in den Aminosäuresequenzen der Δ-6-Desaturase führen, innerhalb einer Population existieren können. Diese genetischen Polymorphismen im Δ-6-Desaturase-Gen können zwischen Individuen innerhalb einer Population aufgrund von natürlicher Variation existieren. Diese natürlichen Varianten bewirken eine Varianz von 1 bis 5 % in der Nukleotidsequenz des Δ-6-Desaturase-Gens. Sämtliche und alle dieser Nukleotidvariationen und daraus resultierende Aminosäurepolymorphismen in der Δ-6-Desaturase, die das Ergebnis natürlicher Variation sind und die funktionelle Aktivität der Δ-6-Desaturase nicht verändern, sollen im Umfang der Erfindung enthalten sein.

Die erfindungsgemäße Nukleinsäuresequenz (oder Fragmente davon können vorteilhaft zur Isolierung weiterer genomischer Sequenzen über Homologiescreening verwendet werden.

Die genannten Derivate lassen sich beispielsweise aus anderen Organismen eukaryontischen Organismen wie Pflanzen wie speziell Moosen, Dinoflagellaten oder Pilze isolieren. Vorteilhaft lassen sich die erfindungsgemäßen Nukleinsäuren und Derivate aus Pflanzen isolieren.

Allelvarianten umfassen insbesondere funktionelle Varianten, die durch Deletion, Insertion oder Substitution von Nukleotiden aus der in SEQ ID NO: 1 dargestellten Sequenz erhältlich sind, wobei die enzymatische Aktivität der abgeleiteten synthetisierten Proteine erhalten bleibt.

Solche DNA-Sequenzen lassen sich ausgehend von den in SEQ ID NO: 1 beschriebenen DNA-Sequenz oder Teilen dieser Sequenzen, beispielsweise mit üblichen Hybridisierungsverfahren oder der PCR-Technik aus anderen Eukaryonten wie beispielsweise den oben genannt isolieren. Diese DNA-Sequenzen hybridisieren unter Standardbedingungen mit den genannten Sequenzen. Zur Hybrisierung werden vorteilhaft kurze Oligonukleotide beispielsweise der konservierten Bereiche, die über Vergleiche mit anderen Desaturasegenen in dem Fachmann bekannter Weise ermittelt werden können, verwendet. Vorteilhaft werden die Histidin-Box-Sequenzen verwendet. Es können aber auch längere Fragmente der erfindungsgemäßen Nukleinsäuren oder die vollständigen Sequenzen für die Hybridisierung verwendet werden. Je nach der verwendeten Nukleinsäure: Oligonukleotid, längeres Fragment oder vollständige Sequenz oder je nachdem welche Nukleinsäureart DNA oder RNA für die Hybridisierung verwendet werden; variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardbedingungen sind beispielsweise je nach Nukleinsäure Temperaturen zwischen 42 und 58°C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 55°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Weiterhin sind unter Derivaten Homologe der Sequenz SEQ ID NO: 1 beispielsweise eukaryontische Homologe, verkürzte Sequenzen, Einzelstrang-DNA der codierenden und nichtcodierenden DNA-Sequenz oder RNA der codierenden und nichtcodierenden DNA-Sequenz zu verstehen.

Außerdem sind unter Homologen der Sequenz SEQ ID NO: 1 Derivate wie beispielsweise Promotorvarianten zu verstehen. Diese Varianten können durch ein oder mehrere Nukleotidaustausche, durch Insertion(en) und/oder Deletion(en) verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremde Organismen ausgetauscht werden.

Unter Derivaten sind auch vorteilhaft Varianten zu verstehen, deren Nukleotidsequenz im Bereich -1 bis -2000 vor dem Startkodon so verändert wurden, dass die Genexpression und/oder die Proteinexpression verändert, bevorzugt erhöht wird. Weiterhin sind unter Derivaten auch Varianten zu verstehen, die am 3'-Ende verändert wurden.

Die erfindungsgemäßen Nukleinsäuresequenzen, die für eine Δ-6-Desaturase kodieren, können synthetisch hergestellt oder natürlich gewonnen sein oder eine Mischung aus synthetischen und natürlichen DNA-Bestandteilen enthalten, sowie aus verschiedenen heterologen Δ-6-Desaturase-Genabschnitten verschiedener Organismen bestehen. Im allgemeinen werden synthetische Nukleotid-Sequenzen mit Codons erzeugt, die von den entsprechenden Wirtsorganismen beispielsweise Pflanzen bevorzugt werden. Dies führt in der Regel zu einer optimalen Expression der heterologen Gene. Diese von Pflanzen bevorzugten Codons können aus Codons mit der höchsten Proteinhäufigkeit bestimmt werden, die in den meisten interessanten Pflanzenspezies exprimiert werden. Ein Beispiel für Corynebacterium glutamicum ist gegeben in: Wada et al. (1992) Nucleic Acids Res. 20:2111-2118). Die Durchführung solcher Experimente sind mit Hilfe von Standardmethoden durchführbar und sind dem Fachmann auf dem Gebiet bekannt.

Funktionell äquivalente Sequenzen, die für die Δ-6-Desaturase-Gene kodieren, sind solche Derivate der erfindungsgemäßen Sequenzen, welche trotz abweichender Nukleotidsequenz noch die gewünschten Funktionen, das heißt die wesentliche enzymatische Aktivität und spezifische Selektivität der Proteine besitzen. Funktionelle Äquivalente umfassen somit natürlich vorkommende Varianten der hierin beschriebenen Sequenzen sowie künstliche, z.B. durch chemische Synthese erhaltene, an den Codon-Gebrauch einer Pflanze angepasste, künstliche Nukleotid-Sequenzen.

Außerdem sind artifizielle DNA-Sequenzen geeignet, solange sie, wie oben beschrieben, die gewünschte Eigenschaft beispielsweise der Erhöhung des Gehaltes von Δ-6-Doppelbindungen in Fettsäuren, Ölen oder Lipiden in der Pflanze durch Überexpression des/der Δ-6-Desaturase-Gens/Gene in Kulturpflanzen vermitteln. Solche artifiziellen DNA-Sequenzen können beispielsweise durch Rückübersetzung mittels Molecular Modelling konstruierter Proteine, Δ-6-Desaturase-Aktivität aufweisen oder durch in vitro-Selektion ermittelt werden. Mögliche Techniken zur in vitro-Evolution von DNA zur Veränderung bzw. Verbesserung der DNA-Sequenzen sind beschrieben bei Patten, P.A. et al., Current Opinion in Biotechnology 8, 724-733(1997) oder bei Moore, J.C. et al., Journal of Molecular Biology 272, 336-347 (1997). Besonders geeignet sind codierende DNA-Sequenzen, die durch Rückübersetzung einer Polypeptidsequenz gemäß der für die Wirtspflanze spezifischen Kodon-Nutzung erhalten werden. Die spezifische Kodon -Nutzung kann ein mit pflanzengenetischen Methoden vertrauter Fachmann durch Computerauswertungen anderer, bekannter Gene der zu transformierenden Pflanze leicht ermitteln.

Als weitere geeignete äquivalente Nukleinsäure-Sequenzen sind zu nennen Sequenzen, welche für Fusionsproteine kodieren, wobei Bestandteil des Fusionsproteins ein Δ-6-Desaturase-Polypeptid oder ein funktionell äquivalenter Teil davon ist. Der zweite Teil des Fusionsproteins kann z.B. ein weiteres Polypeptid mit enzymatischer Aktivität sein oder eine antigene Polypeptidsequenz mit deren Hilfe ein Nachweis auf Δ-6-Desaturase-Expression möglich ist (z.B. myc-tag oder his-tag). Bevorzugt handelt es sich dabei jedoch um eine regulative Proteinsequenz, wie z.B. ein Signalsequenz für das ER, das das Δ-6-Desaturase-Protein an den gewünschten Wirkort leitet.

Vorteilhaft stammen die erfindungsgemäßen isolierten Nukleinsäuresequenzen aus einer Pflanze wie einer monokotylen oder dikotylen Pflanze. Bevorzugt stammen die Nukleinsäuresequenzen aus der Familie der Primulaceae, wie oben beschrieben.

Vorteilhaft können die Δ-6-Desaturase-Gene im erfindungsgemäßen Verfahren mit weiteren Genen der Fettsäurebiosynthese kombiniert werden. Beispiele für derartige Gene sind die Acyltransferasen, weitere Desaturasen oder Elongasen. Für die in vivo und speziell in vitro Synthese ist die Kombination mit z.B. NADH-Cytochrom B5 Reduktasen vorteilhaft, die Reduktionsäquivalente aufnehmen oder abgeben können.

Im Prinzip können alle Gene des Fettsäure- oder Lipidstoffwechsels mit der(den) erfinderischen Δ-6-Desaturase(n) [im Sinne dieser Anmeldung soll der Plural den Singular und umgekehrt beinhalten] zur Herstellung von γ-Linolensäure und/oder Stearidonsäure im Verfahren kombiniert werden vorteilhaft werden Gene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäurn-Elongase(n) verwendet. Besonders bevorzugt werden Gene ausgewählt aus der Gruppe bestehend aus Δ-4-Desaturasen, Δ-5-Desaturasen, Δ-9-Desaturasen, Δ-12-Desaturasen, Δ-6-Elongasen oder Δ-5-Elongasen in Kombination mit den vorgenannten Genen für die Δ-6-Desaturase(n) verwendet, wobei einzelne Gene oder mehrere Gene in Kombination verwendet werden können.

Offenbart under auch Proteine, die durch die erfindungs-gemäßen Nukleinsäuresequenzen codiert werden.

Unter diesen Proteinen (= Polypeptide oder Aminosäuresequenzen) sind Proteine zu verstehen, die eine in der Sequenz SEQ ID NO: 2 dargestellten Aminosäuresequenz oder eine daraus durch Substitution, Inversion, Insertion oder Deletion von einem oder mehreren Aminosäureresten erhältliche Sequenz enthalten, wobei die enzymatische Aktivitäten des in SEQ ID NO: 2 dargestellten Proteins erhalten bleibt bzw. nicht wesentlich reduziert wird. Unter nicht wesentlich reduziert sind alle Enzyme zu verstehen, die noch mindestens 10 %, bevorzugt 20 %, besonders bevorzugt 30 % der enzymatischen Aktivität des Ausgangsenzyms aufweisen. Dabei können beispielsweise bestimmte Aminosäuren durch solche mit ähnlichen physikochemischen Eigenschaften (Raumerfüllung, Basizität, Hydrophobizität etc.) ersetzt werden. Beispielsweise werden Argininreste gegen Lysinreste, Valinreste gegen Isoleucinreste oder Asparaginsäurereste gegen Glutaminsäurereste ausgetauscht. Es können aber auch ein oder mehrere Aminosäuren in ihrer Reihenfolge vertauscht, hinzugefügt oder entfernt werden, oder es können mehrere dieser Maßnahmen miteinander kombiniert werden.

Unter Derivaten sind auch funktionelle Äquivalente zu verstehen, die insbesondere auch natürliche oder künstliche Mutationen einer ursprünglich isolierten für Δ-6-Desaturase codierende Sequenz beinhalten, welche weiterhin die gewünschte Funktion, das heißt deren enzymatische Aktivität und Substratselektivität nicht wesentlich reduziert ist, zeigen. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfasst, welche man durch Modifikation der Δ-6-Desaturase Nukleotidsequenz erhält. Ziel einer solchen Modifikation kann z.B. die weitere Eingrenzung der darin enthaltenen codierenden Sequenz oder z.B. auch die Einfügung weiterer Restriktionsenzym-Schnittstellen sein.

Funktionelle Äquivalente sind auch solche Varianten, deren Funktion, verglichen mit dem Ausgangsgen bzw. Genfragment, abgeschwächt (= nicht wesentlich reduziert) oder verstärkt ist (= Enzymaktivität stärker als die Aktivität des Ausgangsenzym, das heißt Aktivität ist höher als 100 %, bevorzugt höher als 110 %, besonders bevorzugt höher als 130 %).

Die Nukleinsäuresequenz kann dabei vorteilhaft beispielsweise eine DNA- oder cDNA-Sequenz sein. Zur Insertion in ein erfindungsgemäßes Genkonstrukt geeignete codierende Sequenzen sind beispielsweise solche, die für eine Δ-6-Desaturase mit den oben beschriebenen Sequenzen kodieren und die dem Wirt die Fähigkeit zur - Überproduktion von Fettsäuren, Ölen oder Lipiden mit Doppelbindungen in Δ-6-Position verleihen, besonders wenn dabei ω 3 Fettsäuren mit mindestens vier Doppelbindungen hergestellt werden. Diese Sequenzen können homologen oder heterologen Ursprungs sein.

Unter dem erfindungsgemäßen Genkonstrukt (= Expressionskassette, Nukleinsäurekonstrukt oder -fragment) sind die in SEQ ID NO: 1 genannten Sequenzen zu verstehen, die sich als Ergebnis des genetischen Codes ergeben, die mit einem oder mehreren Regulationssignalen vorteilhafterweise zur Erhöhung der Genexpression funktionell verknüpft wurden und welche die Expression der codierenden Sequenz in der Wirtszelle steuern. Diese regulatorischen Sequenzen sollen die gezielte Expression der Gene und der Proteinexpression ermöglichen. Dies kann beispielsweise je nach Wirtspflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können In Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Das Δ-6-Desaturase-Gen kann in einer oder mehreren Kopien in der Expressionskassette (= Genkonstrukt) enthalten sein.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Als Promotoren in der Expressionskassette sind grundsätzlich alle Promotoren geeignet, die die Expression von Fremdgenen in Organismen vorteilhaft in Pflanzen oder Pilzen steuern können. Vorzugsweise verwendet man insbesondere ein pflanzliche Promotoren oder Promotoren, die aus einem Pflanzenvirus entstammen. Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacl^{q-,} T7-, T5-, T3-, gal-, trc-, ara-, SP6-, λ-P_{R}- oder im λ-P_{L}-Promotor enthalten, die vorteilhafterweise in gramnegativen Bakterien Anwendung finden. Weitere vorteilhafte Regulationssequenzen sind beispielsweise in den gram-positiven Promotoren amy und SPO2, in den Hefe-oder Pilzpromotoren ADC1, MFα, AC, P-60, CYC1, GAPDH, TEF, rp28, ADH oder in den Pflanzenpromotoren wie CaMV/35S [Franck et al., Cell 21(1980) 285-294], SSU, OCS, lib4, STLS1, B33, nos (= Nopalin Synthase Promotor) oder im Ubiquitin-Promotor enthalten. Die Expressionskassette kann auch einen chemisch Induzierbaren Promotor enthalten, durch den die Expression des exogenen Δ-6-Desaturase-Gens in den Organismen vorteilhaft in den Pflanzen zu einem bestimmten Zeitpunkt gesteuert werden kann. Derartige vorteilhafte Pflanzenpromotoren sind beispielsweise der PRP1-Promotor [Ward et al., Plant.Mol. Biol.22(1993), 361-366], ein durch Benzensulfonamid-induzierbarer (EP 388186), ein durch Tetrazyklin-induzierbarer (Gatz et al., (1992) Plant J. 2,397-404), ein durch Salizylsäure induzierbarer Promotor (WO 95/19443), ein durch Abscisinsäure-induzierbarer (EP335528) bzw. ein durch Ethanol- oder Cyclohexanon-induzierbarer (WO93/21334) Promotor. Weitere Pflanzenpromotoren sind beispielsweise der Promotor der cytosolischen FBPase aus Kartoffel, der ST-LSI Promotor aus Kartoffel (Stockhaus et al., EMBO J. 8 (1989) 2445-245), der Promotor der Phosphoribosylpyrophosphat Amidotransferase aus Glycine max (siehe auch Genbank Accession Nummer U87999) oder ein Nodien-spezifischen Promotor wie in EP 249676 können vorteilhaft verwandt werden. Vorteilhaft sind insbesonders solche pflanzliche Promotoren, die die Expression in Geweben oder Pflanzenteilen/-organen sicherstellen, in denen die Fettsäurebiosynthese bzw. dessen Vorstufen stattfindet wie beispielsweise im Endosperm oder im sich entwickelnden Embryo. Insbesondere zu nennen sind vorteilhafte Promotoren, die eine samenspezifische Expression gewährleisten wie beispielsweise der USP-Promotor oder Derivate davon, der LEB4-Promotor, der Phaseolin-Promotor oder der Napin-Promotor. Der erfindungsgemäß aufgeführte und besonders vorteilhafte USP-Promotor oder dessen Derivate vermitteln in der Samenentwicklung eine sehr früh Genexpression (Baeumlein et al., Mol Gen Genet, 1991, 225 (3): 459-67). Weitere vorteilhafte samenspezifische Promotoren, die für monokotyle und dikotyle Pflanzen verwendet werden können, sind die für Dikotyle geeignete Promotoren wie ebefalls beispielhaft augeführte Napingen-Promotor aus Raps (US5,608,152), der Oleosin-Promotor aus Arabidopsis (WO98/45461), der Phaseolin-Promotor aus Phaseolus vulgaris (US5,504,200), der Bce4-Promotor aus Brassica (WO91/13980) oder der Leguminosen B4-Promotor (LeB4, Baeumlein et al., Plant J., 2, 2, 1992: 233 - 239) oder für Monokotyle geeignete Promotoren wie die Promotoren die Promotoren des Ipt2- oder Ipt1-Gens aus Gerste (WO95/15389 und WO95/23230) oder die Promotoren des Gersten Hordein-Gens, des Reis Glutelin-Gens, des Reis Oryzin-Gens, des Reis Prolamin-Gens, des Weizen Gliadin-Gens, des Weizen Glutelin-Gens, des Mais Zein-Gens, des Hafer Glutelin-Gens, des Sorghum Kasirin-Gens oder des Roggen Secalin-Gens, die in WO99/16890 beschrieben werden.

Weiterhin sind insbesondere solche Promotoren bevorzugt, die die Expression in Geweben oder Pflanzenteilen sicherstellen, in denen beispielsweise die Biosynthese von Fettsäuren, Ölen und Lipiden bzw. deren Vorstufen stattfindet. Insbesondere zu nennen sind Promotoren, die eine samenspezifische Expression gewährleisten. Zu nennen sind der Promotor des Napin-Gens aus Raps (US 5,608,152), des USP-Promotor aus Vicia faba (USP = unbekanntes Samenprotein, Baeumlein et al., Mol Gen Genet, 1991, 225 (3): 459-67), des Oleosin-Gens aus Arabidopsis (WO98/45461), des Phaseolin-Promotors (US 5,504,200) oder der Promotor des Legumin B4-Gens (LeB4; Baeumlein et al., 1992, Plant Journal, 2 (2): 233-9). Weiterhin sind zu nennen Promotoren, wie der des Ipt2 oder Ipt1-Gens aus Gerste (WO95/15389 und WO95/23230), die in monokotylen Pflanzen samenspezifische Expression vermitteln.

Weitere zur Expression in Pflanzen geeignet Promotoren sind beschrieben (Rogers et al. (1987) Method In Enzymol 153:253-277; Schardl et al. (1987) Gene 61:1-11; Berger et al. (1989) Proc Natl Acad Sci USA 86:8402-8406).

Im Genkonstrukt (= Expressionskassette, Nukleinsäurekonstrukt) können wie oben beschrieben noch weitere Gene, die in die Organismen eingebracht werden sollen, enthalten sein. Diese Gene können unter getrennter Regulation oder unter der gleichen Regulationsregion wie das Δ-6-Desaturase-Gen liegen. Bei diesen Genen handelt es sich beispielsweise um weitere Biosynthesegene vorteilhaft der Fettsäurebiosynthese wie Biosynthesegene des Fettsäure- oder Lipidstoffwechsels, die eine gesteigerte Synthese ermöglichen, ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n). Beispielsweise seien die Gene für die Δ-15-, Δ-12-, Δ-9-, Δ-6-, Δ-5-Desaturase, β-Ketoacyl-Reductasen, β-Ketoacyl-Synthasen, Elongasen oder die verschiedenen Hydroxylasen und Acyl-ACP-Thioesterasen genannt. Vorteilhaft werden Desaturase- und Elongasegene im Nukleinsäurekonstrukt verwendet. Besonders vorteilhaft werden Gene ausgewählt aus der Gruppe Δ-4-Desaturase, Δ-5-Desaturase, Δ-8-Desatuase, Δ-9-Desaturase, Δ-12-Desaturase, Δ-5-Elongase, Δ-6-Elongase oder Δ-9-Elongase im Konstrukt verwendet.

Prinzipiell können alle natürlichen Promotoren mit ihren Regulationssequenzen wie die oben genannten für die erfindungsgemäße Expressionskassette und das erfindungsgemäße Verfahren, wie unten beschrieben, verwendet werden. Darüber hinaus können auch synthetische Promotoren vorteilhaft verwendet werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente (= erfindungsgemäße Nukleinsäuren) miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zum Wirtsorganismus beispielsweise zur Wirtspflanze sein. Die Expressionskassette beinhaltet in der 5'-3'-Transkriptionsrichtung den Promotor, eine DNA-Sequenz, die für ein Δ-6-Desaturase-Gen codiert und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können in vitro-Mutagenese, -primerrepair-, Restriktion oder Ligation verwendet werden. Bei geeigneten Manipulationen, wie z.B. Restriktion, - chewing-back- oder Auffüllen von Überhängen für -bluntends-, können komplementäre Enden der Fragmente für die Ligation zur Verfügung gestellt werden.

Von Bedeutung für eine vorteilhafte hohe Expression kann u.a. das Anhängen des spezifischen ER-Retentionssignals SEKDEL sein (Schouten, A. et al., Plant Mol. Biol. 30 (1996), 781-792), die durchschnittliche Expressionshöhe wird damit verdreifacht bis vervierfacht. Es können auch andere Retentionssignale, die natürlicherweise bei im ER lokalisierten pflanzlichen und tierischen Proteinen vorkommen, für den Aufbau der Kassette eingesetzt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3' der T-DNA (Octopin Synthase) des Ti-Plasmids pTiACH5 entsprechen (Gielen et al., EMBO J.3 (1984), 835 ff) oder entsprechende funktionelle Äquivalente.

Die Herstellung einer Expressionskassette erfolgt durch Fusion eines geeigneten Promotors mit einer geeigneten Δ-6-Desaturase-DNA-Sequenz sowie einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homologe als auch fremdartig bzw. heterolog zur Wirtspflanze sein. Die Expressionskassette beinhaltet in der 5'-3'-Transkriptionsrichtung den Promotor, eine DNA-Sequenz die für ein Δ-6-Desaturase-Gen codiert und eine Region für die transkriptionale Termination. Verschiedene Terminationsbereiche sind gegeneinander beliebig austauschbar.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet in der 5'-3'-Transkriptionsrichtung den Promotor, eine DNA-Sequenz die für ein Δ-6-Desaturase-Gen codiert und eine Region für die transkriptionale Termination. Verschiedene Terminationsbereiche sind gegeneinander beliebig austauschbar.

Die DNA Sequenzen codierend für zwei Δ-6-Desaturasen aus Primula cortusoides oder Primula lutoides beinhaltet alle Sequenzmerkmale, die notwendig sind, um eine dem Ort der Fettsäure-, Lipid- oder Ölbiosynthese korrekte Lokalisation zu erreichen. Daher sind keine weiteren Targetingsequenzen per se notwendig. Allerdings kann eine solche Lokalisation wünschenswert und vorteilhaft sein und daher künstlich verändert oder verstärkt werden, sodass auch solche Fusionskonstrukte eine bevorzugte vorteilhafte Ausführungsform der Erfindung sind.

Insbesondere bevorzugt sind Sequenzen, die ein Targeting in Plastiden gewährleisten. Unter bestimmten Umständen kann auch ein Targeting in andere Kompartimente (referiert: Kermode, Crit. Rev. Plant Sci. 15, 4 (1996), 285-423) z.B. in die Vakuole, in das Mitochondrium, in das Endoplasmatische Retikulum (ER), Peroxisomen, Lipidkörper oder durch ein Fehlen entsprechender operativer Sequenzen ein Verbleib im Kompartiment des Entstehens, dem Zytosol, wünschenswert sein.

Vorteilhafterweise werden die erfindungsgemäßen Nukleinsäuresequenzen zusammen mit mindestens einem Reportergen in eine Expressionskassette kloniert, die in den Organismus über einen Vektor oder direkt in das Genom eingebracht wird. Dieses Reportergen sollte eine leichte Detektierbarkeit über einen Wachstums-, Fluoreszenz-, Chemo-, Biolumineszenz- oder Resistenzassay oder über eine photometrische Messung ermöglichen. Beispielhaft seien als Reportergene Antibiotika-oder Herbizidresistenzgene, Hydrolasegene, Fluoreszenzproteingene, Biolumineszenzgene, Zucker-oder Nukleotidstoffwechselgene oder Biosynthesegene wie das Ura3-Gen, das liv2-Gen, das Luciferasegen, das α-Galactosidasegen, das gfp-Gen, das 2-Desoxyglucose-6-phosphat-Phosphatasegen, das β-Glucuronidase-Gen, β-Lactamasegen, das Neomycinphosphotransferasegen, das Hygromycinphosphotransferasegen oder das BASTA (= Gluphosinatresistenz)-Gen genannt. Diese Gene ermöglichen eine leichte Messbarkeit und Quantifizierbarkeit der Transcriptionsaktivität und damit der Expression der Gene. Damit lassen sich Genomstellen identifizieren, die eine unterschiedliche Produktivität zeigen.

Gemäß einer bevorzugten Ausführungsform umfasst eine Expressionskassette stromaufwärts, d.h. am 5'-Ende der codierenden Sequenz, einen Promotor und stromabwärts, d.h. am 3'-Ende, ein Polyadenylierungssignal und gegebenenfalls weitere regulatorische Elemente, welche mit der dazwischenliegenden codierenden Sequenz für die Δ-6-Desaturase und/oder A-6-Desaturase DNA Sequenz operativ verknüpft sind. Unter einer operativen Verknüpfung versteht man die sequenzielle Anordnung von Promotor, codierender Sequenz, Terminator und ggf. weiterer regulativer Elemente derart, dass jedes der regulativen Elemente seine Funktion bei der Expression der codierenden Sequenz bestimmungsgemäß erfüllen kann. Die zur operativen Verknüpfung bevorzugten Sequenzen sind Targeting-Sequenzen zur Gewährleistung der subzellulären Lokalisation in Plastiden. Aber auch Targeting-Sequenzen zur Gewährleistung der subzellulären Lokalisation im Mitochondrium, im Endoplasmatischen Retikulum (= ER), im Zellkern, in Ölkörperchen oder anderen Kompartimenten sind bei Bedarf einsetzbar sowie Translationsverstärker wie die 5'-Führungssequenz aus dem Tabak-Mosaik-Virus (Gallie et al., Nucl. Acids Res. 15 (1987), 8693 -8711).

Eine Expressionskassette kann beispielsweise einen konstitutiven Promotor (bevorzugt den USP- oder Napin-Promotor), das zu exprimierende Gen und das ER-Retentionssignal enthalten. Als ER-Retentionssignal wird bevorzugt die Aminosäuresequenz KDEL (Lysin, Asparaginsäure, Glutaminsäure, Leucin) verwendet.

Das Genkonstrukt wird zur Expression in einem prokaryontischen oder eukaryontischen Wirtsorganismus beispielsweise einem Mikroorganismus wie einem Pilz oder einer Pflanze vorteilhafterweise in einen Vektor wie beispielsweise einem Plasmid, einem Phagen oder sonstiger DNA inseriert, der eine optimale Expression der Gene im Wirtsorganismus ermöglicht. Geeignete Plasmide sind beispielsweise in E. coli pLG338, pACYC184, pBR-Serie wie z.B. pBR322, pUC-Serie wie pur18 oder pUC19. M113mp-Serie, pKC30, pRep4, pHS1, pHS2, pPLc236, pMBL24, pLG200, pUR290, p1N-III¹¹³-B1, Agt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, weitere vorteilhafte Pilzvektoren werden von Romanos, M.A. et al., [(1992) "Foreign gene expression in yeast: a review", Yeast 8:423-488] und von van den Hondel, C.A.M.J.J. et al. [(1991) "Heterologous gene expression in filamentous fungi] sowie in More Gene Manipulations in Fungi [J.W. Bennet & L.L. Lasure, eds., p. 396-428: Academic Press: San Diego] und in "Gene transfer systems and vector development for filamentous fungi" [van den Hondel, C.A.M.J.J. & Punt, P.J. (1991) in: Applied Molecular Genetics of Fungi, Peberdy, J.F. et al., eds., p. 1-28, Cambridge University Press: Cambridge] beschrieben. Vorteilhafte Hefepromotoren sind beispielsweise 2µM, pAG-1, YEp6, YEp13 oder pEMBLYe23. Beispiele für Algen- oder Pflanzenpromotoren sind pLGV23, pGHlac⁺, pBIN19, pAK2004, pVKH oder pDH51 (siehe Schmidt, R. and Willmitzer, L., 1988). Die oben genannten Vektoren oder Derivate der vorstehend genannten Vektoren stellen eine kleine Auswahl der möglichen Plasmide dar. Weitere Plasmide sind dem Fachmann wohl bekannt und können beispielsweise aus dem Buch Cloning Vectors (Eds. Pouwels P.H. et al. Elsevier, Amsterdam-New York-Oxford, 1985 , ISBN 0 444 904018) entnommen werden. Geeignete pflanzliche Vektoren werden unter anderem in "Methods in Plant Molecular Biology and Biotechnotogy" (CRC Press), Kap. 6/7, S.71-119 beschrieben. Vorteilhafte Vektoren sind sog. shuttle-Vektoren oder binäre Vektoren, die in E. coli und Agrobacterium replizieren.

Unter Vektoren sind außer Plasmiden auch alle anderen dem Fachmann bekannten Vektoren wie beispielsweise Phagen, Viren wie SV40, CMV, Baculovirus, Adenovirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden bevorzugt ist eine chromosomale Replikation.

In einer weiteren Ausgestaltungsform des Vektors kann die erfindungsgemäße Expressionskassette auch vorteilhafterweise in Form einer linearen DNA in die Organismen eingeführt werden und über heterologe oder homologe Rekombination in das Genom des Wirtsorganismus integriert werden. Diese lineare DNA kann aus einem linearisierten Plasmid oder nur aus der Expressionskassette als Vektor oder den erfindungsgemäßen Nukleinsäuresequenzen bestehen.

In einer weiteren vorteilhaften Ausführungsform kann die erfindungsgemäße Nukleinsäuresequenz auch alleine in einen Organismus eingebracht werden.

Sollen neben der erfindungsgemäßen Nukleinsäuresequenz weitere Gene in den Organismus eingeführt werden, so können alle zusammen mit einem Reportergen in einem einzigen Vektor oder jedes einzelne Gen mit einem Reportergen in je einem Vektor in den Organismus eingebracht werden, wobei die verschiedenen Vektoren gleichzeitig oder sukzessive eingebracht werden können.

Der Vektor enthält vorteilhaft mindestens eine Kopie der erfindungsgemäßen Nukleinsäuresequenzen und/oder des erfindungsgemäßen Genkonstruktes.

Beispielhaft kann die pflanzliche Expressionskassette in den Transformationsvektor pRT ((a) Toepfer et al., 1993, Methods Enzymol., 217: 66-78; (b) Toepfer et al. 1987, Nucl. Acids. Res. 15: 5890 ff.) eingebaut werden.

Alternativ kann ein rekombinanter Vektor (= Expressionsvektor) auch in-vitro transkribiert und translatiert werden, z.B. durch Nutzung des T7 Promotors und der T7 RNA Polymerase.

In Prokaryoten verwendete Expressionsvektoren nutzen häufig induzierbare Systeme mit und ohne Fusionsproteinen bzw Fusionsoligopeptiden, wobei diese Fusionen sowohl N-terminal als auch C-terminal oder anderen nutzbaren Domänen eines Proteins erfolgen können. Solche Fusionsvektoren dienen in der Regel dazu: i.) die Expressionsrate der RNA zu erhöhen ii.) die erzielbare Proteinsyntheserate zu erhöhen, iii.) die Löslichkeit des Proteins zu erhöhen, iv.) oder die Reinigung durch einen für die Affinitätschromatographie nutzbare Bindesequenz zu vereinfachen. Häufig werden auch proteolytische Spaltstellen über Fusionsproteine eingeführt, was die Abspaltung eines Teils des Fusionsproteins auch der Reinigung ermöglicht. Solche Erkennungssequenzen für Proteasen erkennen sind z.B. Faktor Xa, Thrombin und Enterokinase.

Typische vorteilhafte Fusions- und Expressionsvektoren sind pGEX [Pharmacia Biotech Inc; Smith, D.B. and Johnson, K.S. (1988) Gene 67: 31-40], pMAL (New England Biolabs, Beverly, MA) and pRIT5 (Pharmacia, Piscataway, NJ) welches Glutathion S-transferase beinhaltet (GST), Maltose Bindeprotein, oder Protein A.

Weitere Beispiele für E. coli Expressionsvektoren sind pTrc [Amann et al., (1988) Gene 69:301-315] und pET Vektoren [Studier et al., Gene Expression Technology: Methods in Enzymology 185, Academic Press, San Diego, California (1990) 60-89; Stratagene, Amsterdam, Niederlande].

Weitere vorteilhafte Vektoren zur Verwendung in Hefe sind pYepSec1 (Baldari, et al., (1987) Embo J. 6:229-234), pMFa (Kurjan and Herskowitz, (1982) Cell 30:933-943), pJRY88 (Schultz et al., (1987) Gene 54:113-123), and pYES-Derivate (Invitrogen Corporation, San Diego, CA). Vektoren für die Nutzung in filamentösen Pilzen sind beschrieben in: van den Hondel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

Alternativ können auch vorteilhaft Insektenzellexpressionsvektoren genutzt werden z.B. für die Expression in Sf 9 Zellen. Dies sind z.B. die Vektoren der pAc Serie (Smith et al. (1983) Mol. Cell Biol. 3:2156-2165) und der pVL series (Lucklow and Summers (1989) Virology 170:31-39).

Des weiteren können zur Genexpression vorteilhaft Pflanzenzellen oder Algenzellen genutzt werden. Beispiele für Pflanzenexpressionsvektoren finden sich in Becker, D., et al. (1992) "New plant binary vectors with selectable markers located proximal to the left border". Plant Mo/. Biol. 20: 1195-1197 oder in Bevan, M.W. (1984) "Binary Agrobacterium vectors for plant transformation", Nucl. Acid. Res. 12: 8711-8721.

Weitere prokaryotische und eukaryotische Expressionssysteme sind genannt in Kapitel 16 und 17 in Sambrook et al., Molecular Cloning: A Laboratory Manual. 2nd, ed., Cold Spring HarborLaboratory, Cold Spring Harbor Laboratory Press, Cold Spring Harbor, NY, 1989.

Das Einbringen der erfindungsgemäßen Nukleinsäuren, der Expressionskassette oder des Vektors in Organismen beispielsweise in Pflanzen kann prinzipiell nach allen dem Fachmann bekannten Methoden erfolgen.

Für Mikroorganismen kann der Fachmann entsprechende Methoden den Lehrbüchern von Sambrook, J. et al. (1989) Molecular cloning: A laboratory manual, Cold Spring Harbor Laboratory Press, von F.M. Ausubel et al. (1994) Current protocols in molecular biology, John Wiley and Sons, von D.M. Glover et al., DNA Cloning Vor.1, (1995), IRL Press (ISBN 019-963476-9), von Kaiser et al. (1994) Methods in Yeast Genetics, Cold Spring Habor Laboratory Press oder Guthrie et al. Guide to Yeast Genetics and Molecular Biology, Methods in Enzymology, 1994, Academic Press entnehmen.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143 sowie in Potrykus Annu. Rev. Plant Physiol. Plant Molec. Biol. 42 (1991) 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984) 8711). Mit einem solchen Vektor transformierte Agrobakterien können dann in bekannter Weise zur Transformation von Pflanzen, insbesondere von Kulturpflanzen, wie z.B. von Tabakpflanzen, verwendet werden, indem beispielsweise verwundete Blätter oder Blattstücke in einer Agrobakterienlösung gebadet und anschließend in geeigneten Medien kultiviert werden. Die Transformation von Pflanzen mit Agrobacterium tumefaciens wird beispielsweise von Höfgen und Willmitzer in Nucl. Acid Res. (1988) 16, 9877 beschrieben oder ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38.

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind wie vorher beschrieben vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes und Übertragung von entsprechenden rekombinanten Ti-Plasmiden oder Ri-Plasmiden durch oder durch Infektion mit transgenen Pflanzenviren durchgeführt werden. Die Agrobacterium-vermittelte Transformation ist am besten für dicotyledone Pflanzenzellen geeignet. Die Verfahren sind beispielsweise beschrieben bei Horsch RB et al. (1985) Science 225: 1229f).

Werden Agrobacterien verwendet, so ist die Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Das Selektionsmarkergen erlaubt eine Selektion transformierter Agrobakteria und ist zum Beispiel das nptll Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA).

Mit einem erfindungsgemäßen Expressionsvektor transformierte Agrobakterien können ebenfalls in bekannter Weise zur Transformation von Pflanzen wie Testpflanzen wie Arabidopsis oder Kulturpflanzen wie Canola, Flachs oder Raps verwendet werden, z.B. indem verwundete Blätter oder Blattstücke in einer Agrobakterienlösung gebadet und anschließend in geeigneten Medien kultiviert werden. Besonders zur Herstellung von γ-Linolensäure und/oder Stearidonsäure eignen sich Pflanzen der Familien der Brassicaceae oder Linaceae. Besonders vorteilhaft eignet sich die Gattung Brassica, Camelina oder Linum zur Herstellung von γ-Linolensäure und/oder Stearidonsäure mit dem erfindungsgemäßen Nukleinsäuresequenzen vorteilhaft in Kombination mit weiteren Desaturasen und Elongasen.

Direkte Transformationstechniken eignen sich für jeden Organismus und Zelltyp. Im Falle von Injektion oder Elektroporation von DNA bzw. RNA in pflanzliche Zellen sind keine besonderen Anforderungen an das verwendete Plasmid gestellt Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G 418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind oben genannt und umfassen bevorzugt das bar Gen, dass Resistenz gegen das Herbizid Phosphinotricin verleiht (Rathore KS et al. (1993) Plant Mol Biol 21 (5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die oben genannten Verfahren sind beispielsweise beschrieben in Jenes B et al.(1993) Techniques for Gene Transfer, in: Transgenic Plants, Vol. 1, Engineering and Utilization, herausgegeben von SD Kung und R Wu, Academic Press, S.128-143 sowie in Potrykus (1991) Annu Rev Plant Physiol Plant Molec Biol 42:205-225). Vorzugsweise wird das zu exprimierende Konstrukt in einen Vektor kloniert, der geeignet ist, Agrobacterium tumefaciens zu transformieren, beispielsweise pBin19 (Bevan et al. (1984) Nucl Acids Res 12:8711f).

Sobald eine transformierte Pflanzenzelle hergestellt wurde, kann eine vollständige Pflanzen unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind such Verfahren bekannt, um aus Pflanzenzellen, Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14:273-278; Jahne et al. (1994) Theor Appl Genet 89:525-533 verwendet. Entsprechende weitere Methoden können den oben genannten Schriften von S.D. Kung und R. Wu, Potrykus oder Höfgen und Willmitzer entnommen werden.

Als transgene Organismen bzw. Wirtsorganismen für die erfindungsgemäße Nukleinsäure, die Genkonstrukte oder den Vektor eignen sich prinzipiell vorteilhaft alle Pflanzen der Familien Brassicaceae oder Linaceae, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Raps, Camelina, Senf oder Flachs genannt. Bevorzugt werden Pflanzen, die natürlicherweise Öle in größeren Mengen synthetisieren können.

Unter transgener Pflanze im Sinne der Erfindung ist zu verstehen, dass die im Verfahren verwendeten Nukleinsäuren nicht an ihrer natürlichen Stelle im Genom der Pflanze sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom der Pflanze sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor.

"Transgen" meint damit beispielsweise bezüglich einer Nukleinsäuresequenz, einem Genkonstrukt oder einem Vektor enthaltend eine Nukleinsäuresequenz, die für die Δ-6-Desaturase oder deren Derivate codiert, oder einem Organismus transformiert mit dieser Nukleinsäuresequenz, einer Expressionskassette oder einem Vektor alle solche durch gentechnische Methoden zustandegekommenen Konstruktionen, in denen sich entweder
a) die Δ-6-Desaturase-Nukleinsäuresequenz, oder
b) eine mit der Δ-6-Desaturase-Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitutionen, Additionen, Deletionen, inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp.

Ein weiterer Gegenstand der Erfindung betrifft die Verwendung einer Expressionskassette enthaltend DNA-Sequenzen codierend für ein Δ-6-Desaturase-Gen oder mit diesen hybridisierende DNA-Sequenzen zur Transformation von Pflanzenzellen, - geweben oder Pflanzenteilen. Ziel der Verwendung ist die Erhöhung des Gehaltes an Fettsäuren, Ölen oder Lipiden mit erhöhtem Gehalt an und Doppelbindungen in Δ-6-Position.

Dabei kann je nach Wahl des Promotors die Expression des Δ-6-Desaturase-Gens spezifisch in den Blättern, in den Samen, den Knollen oder anderen Teilen der Pflanze erfolgen. Solche Fettsäuren, Öle oder Lipide mit Δ-6-Doppelbindungen überproduzierenden transgenen Pflanzen, deren Vermehrungsgut, sowie deren Pflanzenzellen, - gewebe oder-teile, sind ein weiterer Gegenstand der vorliegenden Erfindung. Ein bevorzugter erfindungsgemäßer Gegenstand sind transgene Pflanzen der Familien Brassicaceae oder Linaceae enthaltend eine erfindungsgemäße Nukleinsäuresequenz, ein erfindungsgemäßes Genkonstrukt oder einen erfindungsgemäßen Vektor.

Erhöhung des Gehaltes von Fettsäuren, Ölen oder Lipiden mit Δ-6-Doppelbindungen bedeutet im Rahmen der vorliegenden Erfindung beispielsweise die künstlich erworbene Fähigkeit einer erhöhten Biosyntheseleistung durch funktionelle Überexpression des Δ-6-Desaturase-Gens/der A-6-Desaturase-Gene (im Sinne der Erfindung soll der Singular den Plural mit umfassen und umgekehrt) in den erfindungsgemäßen transgenen Pflanzen gegenüber den nicht gentechnisch modifizierten Ausgangspflanzen zumindest für die Dauer mindestens einer Pflanzengeneration.

Der Biosyntheseort von Fettsäuren, Ölen oder Lipiden beispielsweise ist im allgemeinen der Samen oder Zellschichten des Samens, so dass eine samenspezifische Expression des Δ-6-Desaturase-Gens sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Darüberhinaus ist eine konstitutive Expression des exogenen Δ-6-Desaturase-Gens von Vorteil. Andererseits kann aber auch eine induzierbare Expression wünschenswert erscheinen.

Die Wirksamkeit der Expression des A-6-Desaturase-Gens kann beispielsweise in vitro durch Sproßmeristemvermehrung ermittelt werden. Zudem kann eine in Art und Höhe veränderte Expression des Δ-6-Desaturase-Gens und deren Auswirkung auf die Fettsäure-, Öl- oder Lipidbiosyntheseleistung an Testpflanzen in Gewächshausversuchen getestet werden.

Die Erfindung offenbart zudem:
- Verfahren zur Transformation einer Pflanz aus der Familie der Brassicaceae oder Linaceae dadurch gekennzeichnet, dass man erfindungsgemäße Expressionskassetten enthaltend eine Δ-6-Desaturase-Gensequenz aus Primulaceen oder mit dieser hybridisierende DNA'-Sequenzen in eine Pflanzenzelle, in Kallusgewebe, eine ganze Pflanze oder Protoplasten von Pflanzen einbringt.
- Verwendung einer Δ-6-Desaturase-DNA-Gensequenz oder mit dieser hybridisierende DNA-Sequenzen zur Herstellung von Pflanzen mit erhöhtem Gehalt an Fettsäuren, Ölen oder Lipiden mit Δ-6-Doppelbindungen durch Expression dieser Δ-6-Desaturase DNA-Sequenz in Pflanzen.
- Proteine enthaltend die in SEQ ID NO: 2 dargestellten Aminosäuresequenzen.
- Verwendung der Proteine mit den Sequenzen SEQ ID NO: 2 zur Herstellung von ungesättigten Fettsäuren.

Die Erfindung wird durch die folgenden Beispiele näher erläutert:

### Beispiele

### Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlem in zu exprimierenden Konstrukten sequenziert und überprüft.

### Beispiel 3: Klonierung von PUFA-spezifischen Desaturasen aus Primula cortusoides und Primula lutoides.

Während die Mehrheit der höheren Pflanzen (Angiospermae, Gymnospermae) keine Δ-6-desaturierlen Fettsäuren, den Vorstufen für die Synthese von PUFA, synthetisieren, enthalten die Familien der Boraginaceae, Saxifragaceae und Primulaceae Spezies, die diese Fettsäure akkumulieren. Aus diesem Grund stellen Mitglieder dieser Spezies Quellen zur Identifizierung von Δ-6-Desaturasen dar. *Primula cortusoides* und *Primula lutoides* (Eukaryota; Plantae, Tracheophyta, Angiospermae, Primulales, Primulaceae) wurden ausgewählt, da bei Spezies Δ-6-desaturierte Produkte und hier vor allem 18:4^{Δ6,9,12,15}, der Vorstufe von EPA im Samen akkumulieren. Zur Isolierung von DNA wurden Samen von Chiltern Seeds, Cumbria, England bezogen und unter folgenden Bedingungen angezogen:

Vor der Aussaat wurden die Samen für mindestens 3 Stunden in kaltem Wasser gequollen. Ausgesät wurden die Samen in einem Abstand von mindestens 1 cm auf Blumenerde mit 20 % Sand. Unter der Erde befindet sich eine Schicht aus Perlit (1/3 Volumen zur Erde). Die Samen wurden nach der Aussaat mit Perlit bedeckt, reichlich von oben bewässert und unter Langtagbedingungen (16h Licht, 100 µEm-2s-1, 21 oC, 8 h Nacht, 17°C) gehalten.

GesamtRNA wurde wie in Sayanova et al. 1997, Proc. Natl. Acad. Sci. USA 94, 4211-4216 beschrieben, extrahiert. Erst-Strang cDNA wurde ausgehend von GesamtRNA synthetisiert mittels des SMART RACE cDNA Amplification Kit (Clontech) entsprechend der Herstellerangaben.

Um neue Desaturase-Gene zu identifizieren wurden folgende degenerierte Primer für die Amplification eingesetzt:
Deg 1:
   5'- GGITCA(C/T)GA(T/C) (T/G/A)(C/G)IGGICA(C/T)TA-3'
Deg2:
   5'- CC(A/G)TCIGT(A/G)T(T/G)IA(G/A)IGC(T/C)TCCCA-3-

Folgende Bedingungen wurden für die Amplifikation verwendet

95°C 2 min, gefolgt von 30 Zyklen mit 94°C für 30 s, 30 s mit 55-72°C, 72°C für 2 min. Abschließend wurde mit 10 min bei 72°C verlängert. PCR Amplikons wurden entsprechend der Herstellerangaben in pGEM-T (Promega) kloniert und sequenziert. Die erhaltenen Sequenzangaben wurden zur Herstellung von Voll-Längen klonen eingesetzt. Dazu wurden am 5' und 3' Ende gen-spezfische Primer synthetisiert und ausgehend von der cDNA amplifiziert.

Es wurde je eine Sequenz identifiziert, die Ähnlichkeit zu Desaturase-Genen zeigt.

| Gen | Spezies | Nukleotide | SEQ ID NO: |
|---|---|---|---|
| Cort6 | P. cortusoides | 1353 bp | 3 |
| Lut6 | *P. lutoides* | 1350 bp | 1 |

### Beispiel 4: Klonierung von Expressionsplasmiden zur heterologen Expression von P. cortusoide und P. /utoides Genen in Hefen

Zur heterologen Expression in Hefen wurden über PCR mit entsprechenden spezifischen Primer die entsprechenden Sequenzen amplifiziert und in den Hefe-Expressionsvektor pYES2 (Invitrogen) über *KpnI*-*Sac*I kloniert. Dabei wurden ausschließlich die für die PUFA-Proteine kodierende offenen Leserrahmen der Gene amplifiziert. Zusätzlich wurden am 5' Ende eine Restriktionssequenz für *Kpn*I sowie eine Kozak-Sequenz (Cell 1986, 44:283-292) und am 3' Ende eine Restriktionssequenz für Sacl angehängt:

| Gen | Basenpaare | Primer | SEQ ID NO: |
|---|---|---|---|
| Cort6 | 1353 | Fwd:**GGTACCA**TGGCCAACCCATC**AAAAAA**C | 5 |
| | | Rvs : 3'CCTTCCACACACACGGATAA**GAGC**TCC | 6 |
| Lut6 | 1350 | Fwd: **GGTACCA**TGGCTAACAAATCTC**AAA**C | 7 |
| | | Rvs: 3'CTGTTC**AAA**CTCTCGGGTGA**GGAGCT** | 8 |

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL des 5'-ATG sowie des 3'-Stopp Primers)
0,50 µL Advantage-Polymerase

Die Advantage-Polymerase von Clontech wird eingesetzt.

Reaktionsbedingungen der PCR:
Anlagerungstemperatur: 1 min 55°C
Denaturierungstemperatun 1 min 94°C
Elongationstemperatur. 2 min 72°C
Anzahl der Zyklen: 35

Die PCR Produkte, sowie der pYES2 Vektor wurden mit den Restriktionsenzymen *Kp*nl und *Sac*l 1h bei 37°C inkubiert und die Ligationsreaktion mittels Rapid Ligation Kit(Roche) nach Herstellerangaben durchgeführt. Die Inkubationsreaktionen wurden dann in E. coli DH5α-Zellen (Invitrogen) nach Herstellerangaben transformiert. Positive Klone wurden durch PCR (siehe Reaktion oben) identifiziert und die Plasmid-DNA isoliert (Qiagen Dneasy). Die entstandene Plasmide pYCort6 und pYLut6 wurden durch Sequenzierung überprüft und in den Saccharomyces Stamm W303-1A mittels der Lithiumacetat-Methode transformiert. Zur Kontrolle wurde pYES2 (Leervektor) parallel transformiert. Die Selektion der transformierten Hefen erfolgte auf Komplett-Minimalmedium (CMdum)-Agarplatten mit 2% Glucose, aber ohne Uracil.

Für die Expression der Gene aus *P. cortusoides* und *P*. *lutoides* wurden zunächst Vorkulturen aus jeweils 5 ml CMdum-Flüssigmedium mit 2% (w/v) Raffinose aber ohne Uracil mit je einem ausgewählten Transformanten angeimpft und 2 Tage bei 30°C, 200 rpm inkubiert.

5 ml CMdum-Flüssigmedium (ohne Uracil) mit 2% Raffinose und 300 µM der verschiedener Fettsäuren wurden dann mit den Vorkulturen auf eine OD₆₀₀ von 0,05 angeimpft. Die Expression wurde durch die Zugabe von 2% (w/v) Galactose induziert. Die Kulturen werden für weitere 96 h bei 22°C inkubiert.

### Beispiel 5: Klonierung von Expressionsplasmiden zur Expression in Pflanzen

Für die Transformation von Pflanzen wurden weiterere Transformationsvektoren auf Basis von pBIN19-35S (Bevan M. (1984) Binary Agrobacterium vectors for plant transformation. Nucl. Acids Res. 18:203) erzeugt. Dazu wurden mit folgenden Primerpaaren *Bam*HI *Xba*I-Schniitstellen am 5' und 3'-Ende der kodierenden Sequenz eingefügt:

| Gen | Basenpaare | Primer | SEQ ID NO |
|---|---|---|---|
| Cort6 | 1353 bp | Fwd: **GGATCCA**CCATGGCCAACCCATCAAAAAAC | 9 |
| | | Rvs : 3' CCTTCCACACACACGGAT**AAGGTCTAGA** | 10 |
| Lut6 | 1350 bp | Fwd: **GGATCCA**CCATGGCTAACAAATCTC**AAA**C | 11 |
| | | Rvs : 3' CTGTTC**AAA**CTCTCGGGTGA**GGTCTAGA** | 12 |

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µL Advantage-Polymerase

Die Advantage-Polymerase von Clontech wird eingesetzt.

Die PCR Produkte, sowie der pBin19-35S Vektor wurden mit den Restriktionsenzymen *BamH*I und *Xba*I 1 h bei 37°C inkubiert und die Ligationsreaktion mittels Rapid Ligation Kit(Roche) nach Herstellerangaben durchgeführt. Die Inkubationsreaktionen wurden dann in E. coli DH5α-Zellen (Invitrogen) nach Herstellerangaben transformiert. Positive Klone wurden durch PCR (siehe Reaktion oben) identifiziert und die Plasmid-DNA isoliert (Qiagen Dneasy). Die entstandene Plasmide pBIN-Cort6 und pBIN-Lut6 wurden dann in *Agrobacterium tumefaciens* GC3101 durch Elektroporation transformiert und auf Agar-Platten mit 2% YEB Medium+Kanamycin plattiert. Kanamycin-tolerante Zellen wurden ausgewählt und für die Transformation von *Arabidopsis thaliana* eingesetzt.

### Beispiel 6: Expression von P. cortusoides und P. lutoides Genen in Hefen

Hefen, die wie unter Beispiel 4 mit dem Plasmid pYES2 oder den Plasmiden pYES-Cort6 und pYES-Lut6 transformiert wurden, wurden folgendermaßen analysiert: Die Hefezellen aus den Hauptkulturen wurden durch Zentrifugation (100 x g, 5 min, 20°C) geerntet und mit 100 mM NaHCO_{3,} pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1N methanolischer Schwefelsäure und 2% (v/v) Dimethoxypropan für 1 h bei 80°C inkubiert. Die Extraktion der FAMEs erfolgte durch zweimalige Extraktion mit Petrolether (PE). Zur Entfernung nicht derivatisierter Fettsäuren wurden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert. Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Beispiel 7: Funktionelle Charakterisierung

Die Aktivität und Substratspezifität der einzelnen Gene wurde durch Expression und Fütterung verschiedener Fettsäuren ermittelt. Die Substratspezfität von Desaturasen kann nach Expression in Hefe durch die Fütterung mittels verschiedener Fettsäuren ermittelt werden. Beschreibungen für die Bestimmung der einzelnen Aktivitäten finden sich in WO 93111245, WO 94/11516, WO 93106712, US 5,614,393, US5614393, WO 96/21022, WO0021557 und WO 99/27111, Qiu et al. 2001, J. Biol. Chem. 276, 31561-31566 für Δ-4-Desaturasen, Hong et al. 2002, Lipids 37,863-868 für Δ5-Desaturasen.

### a) Charakterisierung von Cort6:

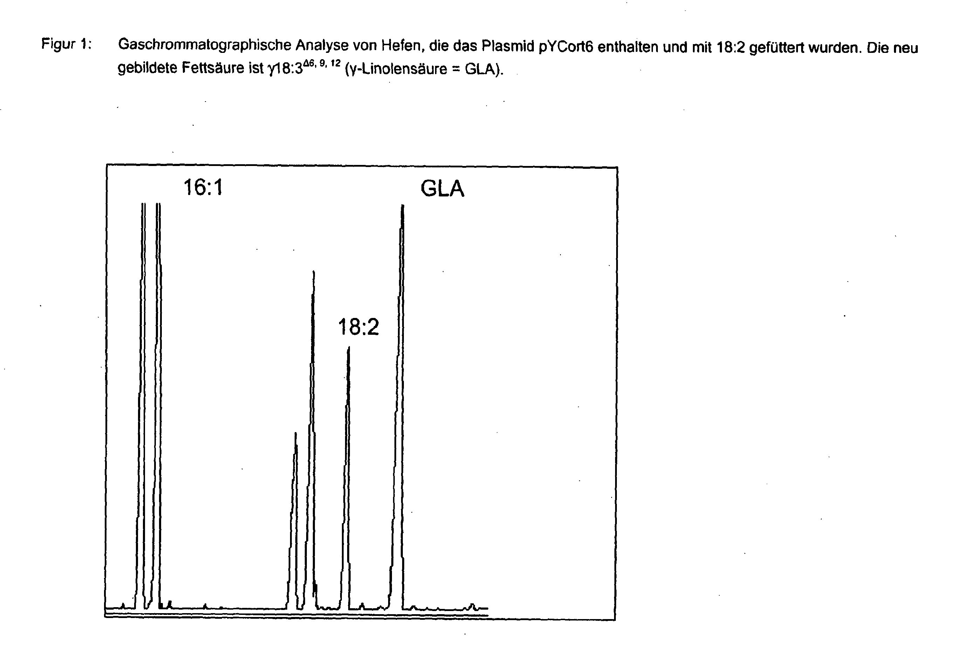

Das Konstrukt pYES-Cort6 wurde durch Fütterung mit verschiedenen Fettsäuren in Hefe getestet Überraschenderweise konnte in diesem Experiment gezeigt werden, dass nach Fütterung mit den Fettsäuren 18:2^{Δ9, 12} und 18:3^{Δ9, 12, 15} neue Fettsäuren entstanden sind (Figur 1).

Figur 1: Gaschrommatographische Analyse von Hefen, die das Plasmid pYCort6 enthalten und mit 18:2 gefüttert wurden. Die neu gebildete Fettsäure ist γ18:3^{Δ6, 9, 12} (γ-Linolensäure = GLA).

Auf Grund der neu entstandenen Fettsäure konnte für Cort6 eine Δ-6-Desaturaseaktivität nachgewiesen werden. Das Enzym konnte dabei 18:2 und 18:3 als Substrat verwerten.

### b) Charakterisierung von Lut6:

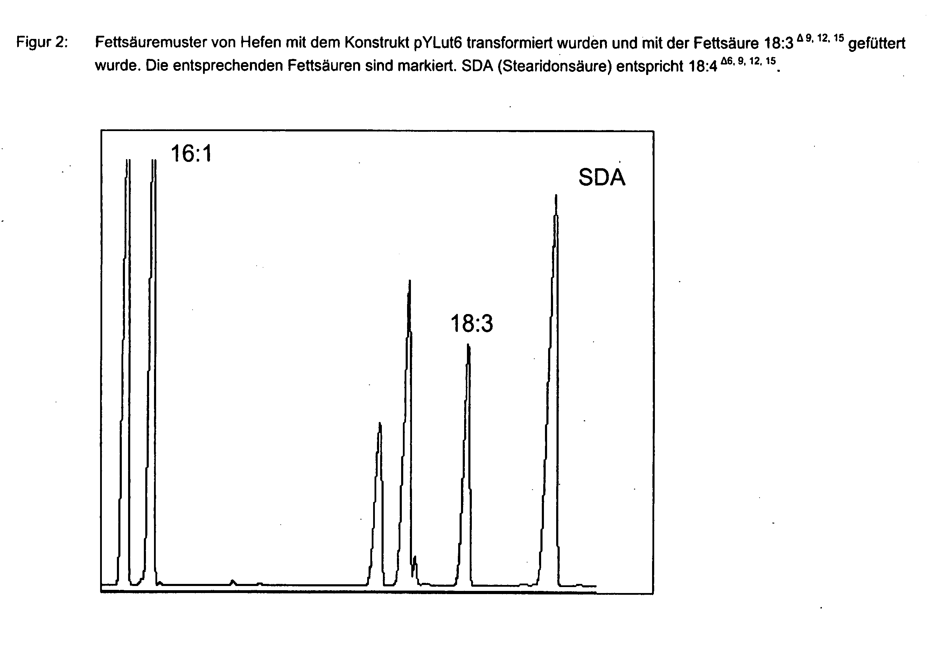

Überraschenderweise konnte nach Fütterung von 18:3 gezeigt werden, dass Lut6 eine Δ-6-Desaturase-Aktivität besitzt (Figur 2).

Figur 2: Fettsäuremuster von Hefen mit dem Konstrukt pYLut6 transformiert wurden und mit der Fettsäure 18:3^{Δ9, 12, 15} gefüttert wurde. Die entsprechenden Fettsäuren sind markiert. SDA (Stearidonsäure) entspricht 18:4^{Δ6, 9, 12, 15}

Die Aktivität von Lut6 entspricht damit der Aktivität einer Δ-6-Desaturasen.

### Beispiel 8: Erzeugung von transgenen Pflanzen

### a) Erzeugung transgener Rapspflanzen (verändert nach Moloney et al., 1992, Plant Cell Reports, 8:238-242)

Zur Erzeugung transgener Rapspflanzen werden binäre Vektoren wie die unter Beispiel 5 erzeugten pBIN-35S Plasmide in *Agrobacterium tumefaciens* GC3101 transformiert (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Co-Inkubation in Dunkelheit bei 25°C auf 3MS-Medium mit 0,8 % Bacto-Agar. Die Kultivierung wurde nach 3 Tagen mit 16 Stunden Licht /8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Expression der Desaturase- bzw. Elongase-Gene mittels Lipidanalysen untersucht wie beispielhaft in Qiu et al. 2001, J. Biol. Chem. 276, 31561-31566 beschrieben.

### b) Herstellung von transgenen Leinpflanzen

Die Herstellung von transgenen Leinpflanzen können zum Beispiel nach der Methode von Bell et al., 1999, In Vitro Cell. Dev. Biol-Plant 35(6):456-465 mittels particle bombartment erzeugt werden. Agrobakterien-vermittelte Transformationen können zum Beispiel nach Mlynarova et al. (1994), Plant Cell Report 13: 282-285 hergestellt werden

### Beispiel 9: Lipidextraktion aus Blättern:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX. 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Ein Beispiel ist die Analyse von Fettsäuren (Abkürzungen: FAME, Fettsäuremethylester, GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen. Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1 h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Analyse von trangenen Arabidopsis Pflanzen, die Cort6 exprimieren:
Pflanze, die wie in Beispiel 5 mit den Plasmiden pBIN-Cort6 und pBIN-Lut6 transformiert wurden, wurden auf veränderte Fettsäuren untersucht. Als Ausgangsmaterial wurden Blätter eingesetzt, die entsprechend obiger Beschreibung extrahiert und gaschromatographisch analysiert wurden.

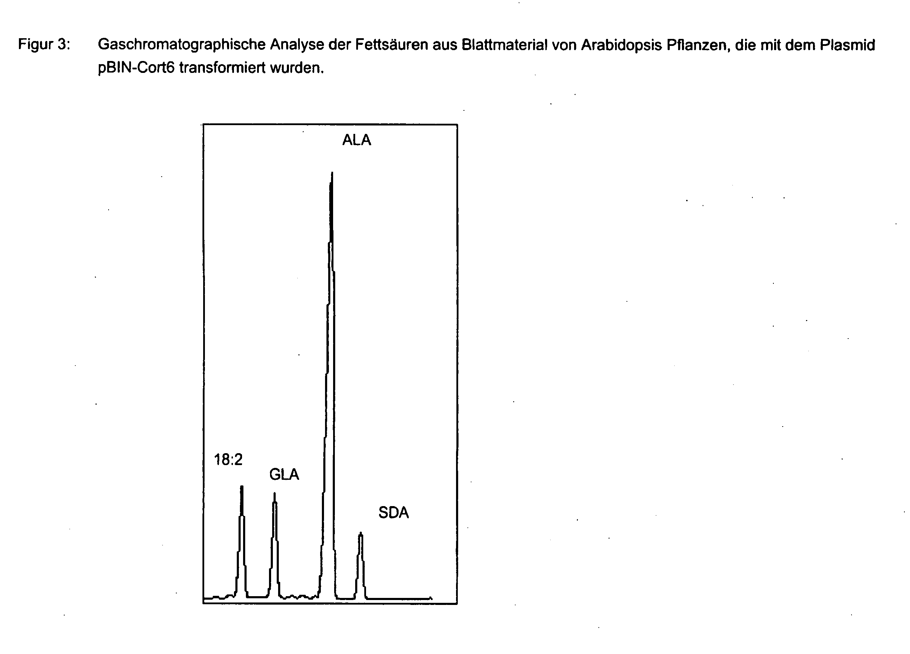

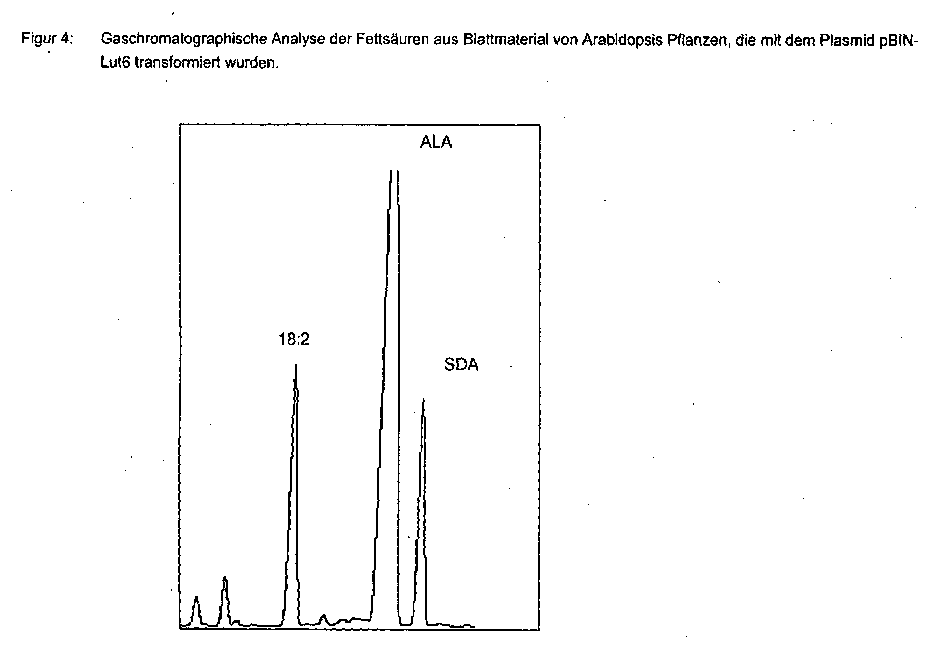

Für beide Plasmide konnte dabei die Entstehung neuer Fettsäuren gezeigt werden (Figuren 3 und 4). Dabei konnte gezeigt werden, dass das Enzym Cort6 vorzugsweise die endogen vorhandenen Fettsäure 18:2^{Δ9, 12} umsetzt, während Lut6 überraschenderweise 18:3^{Δ9, 12, 15} zu SDA (18:4^{Δ6, 9, 12, 15}) bevorzugt umsetzt. Daraus kann geschlossen werden, dass die beiden Enzyme im pflanzlichen Hintergrund bevorzugt zur Herstellung von GLA bzw. SDA, Vorstufen der PUFA ARA bzw. EPA, eingesetzt werden können.

Figur 3: Gaschromatographische Analyse der Fettsäuren aus Blattmaterial von Arabidopsis Pflanzen, die mit dem Plasmid pBIN-Cort6 transformiert wurden.

## Patentansprüche

1. Verfahren zur Herstellung von γ-Linolensäure (18:3 ^{Δ6,9.12}) oder Stearidonsäure (18:4 ^{Δ6.9.12.15}) oder γ-Linolensäure (18:3 ^{Δ6.9.12}) und Stearidonsäure (18:4 ^{Δ6.9.12.15}) in transgenen Pflanzen der Familie Brassicaceae oder Linaceae, **dadurch gekennzeichnet, dass** die transgenen Pflanzen mindestens 10 Gew-% Ölsäure bezogen auf den Gesamtfettsäuregehalt enthalten und das folgende Verfahrensschritte umfassend:
a) Einbringen einer Nukleinsäuresequenz in die Ölpflanze, die für eine Δ-6-Desaturase aus einer Primula-Art codiert und als Substrat α-Linolensäure gegenüber Linolsäure bevorzugt, und
b) Expression der durch die Nukleinsäure codierten Δ-6-Desaturase- in der transgenen Pflanze, und
c) Anzucht und Ernte der Pflanzen,
**dadurch gekennzeichnet, dass** die Nukleinsäuresequenz, die für eine Δ-6-Desaturase aus einer Primula-Art codiert, ausgewählt ist aus der Gruppe bestehend aus:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide codieren, die auf Aminosäureebene mindestens 95 % Identität mit SEQ ID NO: 2 aufweisen und eine Δ-6-Desaturaseaktivität haben,
wobei die D-6-Desaturase durch eine Substratspezifität gegenüber α-Linolensäure, die um mehr als 20fach höher ist als die Substratspezifität gegenüber Linolsäure charakterisiert ist.

2. Verfahren nach Anspruch 1, **dadurch gekennzeichnet, dass** für die Expression der Nukleinsäuresequenzen gemäß Anspruch 1 (a), (b) und (c) diese funktionell mit einem Promotor oder Terminator oder Promotor und Terminator verbunden sind.

3. Verfahren nach Anspruch 1 oder 2, **dadurch gekennzeichnet, dass** die Δ-6-Desaturaseaktivität zu einem erhöhten Gehalt an Δ-6-C₁₈-Fettsäuren in den Pflanzen führt.

4. Verfahren nach einem der Ansprüche 1 bis 3, **dadurch gekennzeichnet, dass** die als A-6-Desaturase als Substrat nur α-Linolensäure akzeptiert und Linolsäure nicht umgesetzt wird.

5. Verfahren nach einem der Ansprüche 1 bis 4, **dadurch gekennzeichnet, dass** ungesättigten Fettsäuren γ-Linolensäure (18:3 ^{Δ6,9,12}) oder (18:4 ^{Δ6,9,12,15}) oder y-Linolensäure (18:3 ^{Δ,6,9,12}) und (18:4 ^{Δ,6,9,12,15}) im Samen der Ölpflanze erhöht werden.

6. Verfahren nach einem der Ansprüche 1 bis 5, **dadurch gekennzeichnet, dass** aus den geernteten Pflanzen die Samen isoliert werden.

7. Verfahren nach den Ansprüchen 1 bis 6, **dadurch gekennzeichnet, dass** die ungesättigten Fettsäuren γ-Linolensäure (18:3 ^{Δ6,9,12}) oder (18:4 ^{Δ6,9,12,15}) oder γ-Linolensäure (18:3 ^{Δ6,9,12}) und (18:4 ^{Δ6,9,12,15}) in Form eines Öls, Lipids oder in Form der freien Fettsäuren aus den transgenen Pflanzen der Familie Brassicaceae oder deren Samen isoliert werden.

8. Verfahren nach Anspruch 7, **dadurch gekennzeichnet, dass** die im Öl oder in den Lipiden enthaltenen ungesättigten Fettsäuren in Form der freien Fettsäuren isoliert werden.

9. Verfahren nach den Ansprüchen 1 bis 8, **dadurch gekennzeichnet, dass** die Öle, Lipide oder freien Fettsäuren mit andern tierischen, mikrobiellen oder pflanzlichen Ölen, Lipiden oder Fettsäuren zu Fettsäurezusammensetzungen gemischt werden.

10. Verfahren nach den Ansprüchen 1 bis 9, **dadurch gekennzeichnet, dass** die Öle, Lipide oder freien Fettsäuren oder Fettsäurezusammensetzungen Futtermitteln, Nahrungsmitteln, Kosmetika oder Pharmazeutika zugesetzt werden.

11. Isolierte Nukleinsäuresequenzen, die für Polypeptide mit Δ-6-Desaturaseaktivität codieren und wobei die codierte Δ-6-Desaturase als Substrat α-Linolensäure gegenüber Linolsäure bevorzugt, ausgewählt aus der Gruppe bestehend aus:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 2 dargestellten Aminosäuresequenz ableiten lassen, oder
c) Derivate der in SEQ ID NO: 1 dargestellten Nukleinsäuresequenz, die für Polypeptide codieren, die auf Aminosäureebene mindestens 95 % Identität mit SEQ ID NO: 2 aufweisen und eine Δ-6-Desaturaseaktivität haben,
wobei die D-6-Desaturase durch eine Substratspezifität gegenüber α-Linolensäure, die um mehr als 20fach höher ist als die Substratspezifität gegenüber Linolsäure charakterisiert ist.

12. Proteine, die von einer isolierten Nukleinsäuresequenz nach Anspruch 11 codiert wird.

13. Genkonstrukt, enthaltend eine isolierte Nukleinsäure nach Anspruch 11, wobei die Nukleinsäure funktionsfähig mit einem oder mehreren Regulationssignalen verbunden ist.

14. Genkonstrukt nach Anspruch 13, **dadurch gekennzeichnet, dass** das Nukleinsäurekonstrukt zusätzliche Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n).

15. Genkonstrukt nach Anspruch 13 oder 14, **dadurch gekennzeichnet, dass** das Nukleinsäurekonstrukt zusätzliche Biosynthesegene des Fettsäure- oder Lipidstoffwechsels enthält ausgewählt aus der Gruppe der A-4-Desaturasen, Δ-5-Desaturasen, Δ-8-Desatuasen, Δ-9-Desaturasen, Δ-12-Desaturasen, Δ-5-Elongasen, Δ-6-Elongasen oder Δ-9-Elongasen.

16. Vektor, enthaltend eine Nukleinsäure nach Anspruch 11 oder ein Genkonstrukt nach Anspruch 13 bis 15.

17. Transgene Brassicaceae oder Linaceae enthaltend als Transgen mindestens eine Nukleinsäure nach Anspruch 11, wobei sich die besagte Nukleinsäur, die für die Δ-6-Desaturase- kodiert, nicht in ihrer natürlichen genetischen Umgebung befindet oder durch gentechnische Methoden modifiziert wurde, ein Genkonstrukt nach Anspruch 13 oder 15 oder einen Vektor nach Anspruch 16.

## Claims

1. A method of producing γ-linolenic acid (18:3 ^{Δ6,9,12}) or stearidonic acid (18:4 ^{Δ6,9,12,15}) or γ-linolenic acid (18:3 ^{Δ6,9,12}) and stearidonic acid (18:4 ^{Δ6,9,12,15}) in transgenic plants of the family Brassicaceae or Linaceae, wherein the transgenic plants comprise at least 10% by weight of oleic acid based on the total fatty acid content and which comprises the following process steps:
a) introducing a nucleic acid sequence into the oil plant which codes for a Δ6-saturase from a Primula species and which preferentially utilizes as substrate, α-linolenic acid to linolenic acid, and
b) expression, in the transgenic plant, of the Δ6-desaturase encoded by the nucleic acid, and
c) growing and harvesting the plants,
wherein the nucleic acid sequence which codes for a Δ6-desaturase from a Primula species is selected from the group consisting of:
a) a nucleic acid sequence with the sequence shown in SEQ ID NO: 1,
b) nucleic acid sequences which, as the result of the degeneracy of the genetic code, can be derived from the amino acid sequence shown in SEQ ID NO: 2, or
c) derivatives of the nucleic acid sequence shown in SEQ ID NO: 1 which code for polypeptides which have at least 95% identity at the amino acid level with SEQ ID NO: 2 and which have a Δ6-desaturase activity,
the D6-desaturase being **characterized by** a substrate specificity for α-linolenic acid which is more than 20 times higher than the substrate specificity for linoleic acid.

2. The method according to claim 1, wherein, to express the nucleic acid sequences according to claim 1 (a), (b) and (c), the latter are linked operably with a promoter or terminator or promoter and terminator.

3. The method according to claim 1 or 2, wherein the Δ6-desaturase activity leads to an increased Δ6-C₁₈-fatty acid content in the plants.

4. The method according to any of claims 1 to 3, wherein the Δ6-desaturase accepts only α-linolenic acid as its substrate and linoleic acid is not converted.

5. The method according to any of claims 1 to 4, wherein unsaturated fatty acids y-linolenic acid (18:3 ^{Δ6,9,12}) or (18:4 ^{Δ6,9,12,15}) or γ-linolenic acid (18:3 ^{Δ6,9,12}) and (18:4 ^{Δ6,9,12,15}) are increased in the seed of the oil plant.

6. The method according to any of claims 1 to 5, wherein the seeds are isolated from the harvested plants.

7. The method according to claims 1 to 6, wherein the unsaturated fatty acids y-linolenic acid (18:3 ^{Δ6,1,12}) or (18:4 ^{Δ6,9,12,15}) or γ-linolenic acid (18:3 ^{Δ6.9,12}) and (18:4 ^{Δ6,9,12,15}) are isolated in the form of an oil, lipid or in the form of the free fatty acids from the transgenic plants of the family Brassicaceae or their seeds.

8. The method according to claim 7, wherein the unsaturated fatty acids which are present in the oil or in the lipids are isolated in the form of the free fatty acids.

9. The method according to claims 1 to 8, wherein the oils, lipids or free fatty acids are mixed with other animal, microbial or plant oils, lipids or fatty acids to give fatty acid compositions.

10. The method according to claims 1 to 9, wherein the oils, lipids or free fatty acids or fatty acid compositions are added to foods, feeds, cosmetics or pharmaceuticals.

11. An isolated nucleic acid sequence which codes for a polypeptide with Δ6-desaturase activity and where the encoded Δ6-desaturase preferentially utilizes, as substrate, α-linolenic acid to linoleic acid, selected from the group consisting of:
a) a nucleic acid sequence with the sequence shown in SEQ ID NO: 1,
b) nucleic acid sequences which, as the result of the degeneracy of the genetic code, can be derived from the amino acid sequence shown in SEQ ID NO: 2, or
c) derivatives of the nucleic acid sequence shown in SEQ ID NO: 1 which code for polypeptides which have at least 95% identity at the amino acid level with SEQ ID NO: 2 and which have a Δ6-desaturase activity,
the D6-desaturase being **characterized by** a substrate specificity for α-linolenic acid which is more than 20 times higher than the substrate specificity for linoleic acid.

12. A protein which is encoded by an isolated nucleic acid sequence according to claim 11.

13. A gene construct comprising an isolated nucleic acid according to claim 11, the nucleic acid being linked operably with one or more regulatory signals.

14. The gene construct according to claim 13, wherein the nucleic acid construct comprises additional biosynthetic genes of the fatty acid or lipid metabolism selected from the group consisting of acyl-CoA dehydrogenase(s), acyl-ACP[= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenases, lipoxygenases, triacylglycerol lipases, allene oxide synthases, hydroperoxide lyases or fatty acid elongase(s).

15. The gene construct according to claim 13 or 14, wherein the nucleic acid construct comprises additional biosynthetic genes of the fatty acid or lipid metabolism selected from the group consisting of the Δ4-desaturases, Δ5-desaturases, Δ8-desaturases, Δ9-desaturases, Δ12-desaturases, Δ5-elongases, Δ6-elongases or Δ9-elongases.

16. A vector comprising a nucleic acid according to claim 11 or a gene construct according to claims 13 to 15.

17. A transgenic Brassicacea or Linacea, comprising, as transgene, at least one nucleic acid according to claim 11, said nucleic acid which codes for the Δ6-desaturase not being situated in its natural genetic environment or having been modified by genetic engineering methods, a gene construct according to claim 13 or 15 or a vector according to claim 16.

## Revendications

1. Procédé de préparation d'acide γ-linolénique (18:3^{Δ6,9,12}) ou d'acide stéaridonique (18:4^{Δ6,9,12,15}) ou d'acide γ-linolénique (18:3^{Δ6,9,12}) et d'acide stéaridonique (18:4^{Δ6,9,12,15}) dans des plantes transgéniques de la famille des Brassicaceae ou des Linaceae, **caractérisé en ce que** les plantes transgéniques contiennent au moins 10 % en poids d'acide oléique par rapport à la teneur totale en acides gras, et comprenant les étapes suivantes
a) introduction d'une séquence d'acides nucléiques dans la plante oléagineuse, qui code pour une Δ-6-désaturase d'une espèce de Primula, et qui en tant que substrat préfère l'acide α-linolénique à l'acide linoléique, et
b) expression de la Δ-6-désaturase codée par l'acide nucléique dans la plante transgénique, et
c) culture et récolte des plantes,
**caractérisé en ce que** la séquence d'acides nucléiques qui code pour une Δ-6-désaturase d'une espèce de Primula est choisie dans le groupe consistant en a) une séquence d'acides nucléiques ayant la séquence présentée dans SEQ ID N°1,
b) des séquences d'acides nucléiques qui peuvent dériver, en tant que résultat du code génétique dégénéré, de la séquence d'acides aminés présentée dans SEQ ID N°2, ou
c) des dérivés de la séquence d'acides nucléiques présentée dans SEQ ID N°1, qui codent pour des polypeptides qui, au niveau des acides aminés, présentent une identité d'au moins 95 % avec SEQ ID N°2 et ont une activité de Δ-6-désaturase,
la Δ-6-désaturase étant **caractérisée par** une spécificité de substrat vis-à-vis de l'α-linolénique qui est de plus de 20 fois supérieure à la spécificité de substrat vis-à-vis de l'acide linoléique.

2. Procédé selon la revendication 1, **caractérisé en ce que**, pour l'expression des séquences d'acides nucléiques selon la revendication 1(a), (b) et (c), ces dernières sont liées d'une manière fonctionnelle à un promoteur ou à un terminateur, ou à un promoteur et à un terminateur.

3. Procédé selon la revendication 1 ou 2, **caractérisé en ce que** l'activité de Δ-6-désaturase conduit dans les plantes à une teneur plus élevée en acides gras en Δ-6-C₁₈.

4. Procédé selon l'une des revendications 1 à 3, **caractérisé en ce que** la Δ-6-désaturase, seul est accepté en tant que substrat l'acide α-linolénique, l'acide linoléique n'étant pas converti.

5. Procédé selon l'une des revendications 1 à 4, **caractérisé en ce que** les acides gras insaturés acide γ-linolénique (18:3^{Δ6,9,12}) ou (18:4^{Δ6,9,12,15}) ou acide γ-linolénique (18:3^{Δ6,9,12} et (18:4^{Δ6,9,12,15}) sont augmentés dans la graine de la plante oléagineuse.

6. Procédé selon l'une des revendications 1 à 5, **caractérisé en ce que** les graines sont isolées des plantes récoltées.

7. Procédé selon l'une des revendications 1 à 6, **caractérisé en ce que** les acides gras insaturés acide γ-linolénique (18:3^{Δ6,9,12}) ou (18:4^{Δ6,9,12,15}) on acide γ-linolénique (18:3^{Δ6,9,12}) et (18:4^{Δ6, 9,12,15}) sont isolés sous forme d'une huile, d'un lipide, ou sous forme des acides gras libres, à partir des plantes transgéniques de la famille des Brassicaceae, ou de leurs graines.

8. Procédé selon la revendication 7, **caractérisé en ce que** les acides gras insaturés contenus dans l'huile ou dans les lipides sont isolés sous forme des acides gras libres.

9. Procédé selon les revendications 1 à 8, **caractérisé en ce que** les huiles, les lipides et les acides gras libres sont mélangés à d'autres huiles, lipides ou acides gras animaux, microbiens ou végétaux, pour donner des compositions d'acides gras.

10. Procédé selon les revendications 1 à 9, **caractérisé en ce que** les huiles, les lipides ou les acides gras libres ou les compositions d'acides gras libres sont ajoutés à des produits pour l'alimentation animale, des produits alimentaires, des produits cosmétiques ou pharmaceutiques.

11. Séquences isolées d'acides nucléiques qui codent pour des polypeptides ayant une activité de Δ-6-désaturase, la Δ-6-désaturase codée préférant en tant que substrat l'acide α-linolénique à l'acide linoléique, choisies dans le groupe consistant en
a) une séquence d'acides nucléiques ayant la séquence présentée dans SEQ ID N°1,
b) des séquences d'acides nucléiques qui peuvent dériver, en tant que résultat du code génétique dégénéré, de la séquence d'acides aminés présentée dans SEQ ID N°2, ou
c) les dérivés de la séquence d'acides nucléiques présentée dans SEQ ID N°1, qui codent pour des polypeptides qui, au niveau des acides aminés, présentent une identité d'au moins 95 % avec SEQ ID N°2 et ont une activité de Δ-6-désaturase,
la Δ-6-désaturase étant **caractérisée par** une spécificité de substrat vis-à-vis de l'α-linolénique qui est de plus de 20 fois supérieure à la spécificité de substrat vis-à-vis de l'acide linoléique.

12. Protéines qui sont codées par une séquence isolée d'acides nucléiques selon la revendication 11.

13. Gène chimère contenant un acide nucléique isolé selon la revendication 11, l'acide nucléique étant lié d'une manière fonctionnelle à un ou plusieurs signaux de régulation.

14. Gène chimère selon la revendication 13, **caractérisé en ce que** la construction d'acide nucléique contient des gènes de biosynthèse supplémentaires du métabolisme des acides gras ou des lipides, choisis dans le groupe comprenant la ou les acyl-CoA-déshydrogénases, la ou les acyl-ACP(= protéine transporteur de groupements acyle)-désaturases, la ou les acyl-ACP-thioestérases, la ou les acide gras-acyl-transférases, la ou les acyl-CoA:lysophospholipide-acyltransférases, la ou les acide gras-synthases, la ou les acide gras-hydroxylases, la ou les acétyl-coenzyme-A-carboxylases, la ou les acyl-coenzyme A-oxydases, la ou les acide gras-désaturases, les acides gras-acétylénases, les lipoxygénases, les triacylglycérol-lipases, les allène-oxyde-synthases, les hydroperoxyde-lyases ou la ou les acide gras-élongases.

15. Gène chimère selon la revendication 13 ou 14, **caractérisé en ce que** la construction d'acide nucléique contient des gènes de biosynthèse supplémentaires du métabolisme des acides gras ou des lipides, choisis dans le groupe des Δ-4-désaturases, des Δ-5-désaturases, des Δ-8-désaturases, des Δ-9-désaturases, des Δ-12-désaturases, des Δ-5-élongases, des Δ-6-élongases ou des Δ-9-élongases.

16. Vecteur contenant un acide nucléique selon la revendication 1.1 ou un gène chimère selon les revendications 13 à 15.

17. Brassicaceae ou Linaceae transgéniques contenant en tant que transgène au moins un acide nucléique selon la revendication 11, ledit acide nucléique, qui code pour la Δ-6-désaturase, ne se trouvant pas dans son environnement génétique naturel, ou ayant été modifié par des méthodes de modification génétique, un gène chimère selon la revendication 13 ou 15 ou un vecteur selon la revendication 16.
